# EUROPEAN PATENT APPLICATION

(11) **EP 4 752 685 A1**
(43) Date of publication of application: **03.06.2026**
(21) Application number: 24858587.9
(22) Date of filing: 28.08.2024
(51) Int. Cl.: G06F 3/01, A61B 5/374, A61B 5/375

(54) **SIGNAL PROCESSING METHOD AND RELATED APPARATUS**

(30) Priority: 03.09.2023 CN 202311133501
(71) Applicant: HUAWEI TECHNOLOGIES CO., LTD., Shenzhen 518129 (CN)
(72) Inventor: TIAN, Kun, Shenzhen, Guangdong 518129 (CN); CHEN, Yun, Shenzhen, Guangdong 518129 (CN); HUANG, Heming, Shenzhen, Guangdong 518129 (CN); LIAO, Jianxing, Shenzhen, Guangdong 518129 (CN); WANG, Tieying, Shenzhen, Guangdong 518129 (CN); ZHANG, Jun, Shenzhen, Guangdong 518129 (CN)
(74) Representative: Epping - Hermann - Fischer
(86) International application number: PCT/CN2024/114968
(87) International publication number: WO 2025/045053

(57) **Abstract**

This application provides a signal processing method. The method is applied to the field of brain-computer interface (BCI) technologies, and includes: obtaining a first signal, where the first signal is a signal acquired from a brain tissue; obtaining a downstream feedback signal; and processing the first signal based on the feedback signal. In the method, the first signal acquired from the brain tissue is processed based on the feedback signal, instead of directly inputting the first signal into a decoding apparatus for decoding, thereby ensuring long-term stability of signal encoding and decoding, avoiding deterioration or even failure, in long-term stability of signal encoding and decoding, that is caused due to signal non-stationarity, and meeting a service requirement.

## Description

This application claims priority to Chinese Patent Application No. 202311133501.2, filed with the China National Intellectual Property Administration on September 3, 2023 and entitled "SIGNAL PROCESSING METHOD AND RELATED APPARATUS", which is incorporated herein by reference in its entirety.

### TECHNICAL FIELD

This application relates to the field of artificial intelligence (artificial intelligence, AI) technologies, and in particular, to a signal processing method and apparatus, a chip, a system, a computer-readable storage medium, and a computer program product.

### BACKGROUND

In recent years, a brain-computer interface (Brain-Computer Interface, BCI) has gradually become an important enabling technology for a future intelligent world. For example, in the medical field, a neurosurgical robot is used to implant a proprietary technology chip and an information bar into a brain tissue, and then a brain signal may be directly read through a universal serial bus (Universal Serial Bus, USB) interface. This method features a minor injury to the brain tissue and a wide application prospect.

The BCI is a new communication and control technology that is developed between the brain tissue (for example, a human brain or an animal brain) and a computer or another electronic device and that does not depend on a conventional brain information output path (an external nerve and a muscle tissue). BCls are classified, based on implantation degrees of an electrode, into two types: an intrusive BCI and a non-invasive BCI.

Both a signal acquired in an intrusive manner and a signal acquired in a non-intrusive manner are non-stationary signals. Specifically, the signals are greatly affected by factors such as electrode deactivation, a random noise, a motion artifact, and a physical and mental status. Consequently, deterioration or even failure in long-term stability of signal encoding and decoding is caused, and a service requirement can hardly be met.

### SUMMARY

This application provides a signal processing method. In the method, a first signal acquired from a brain tissue is processed based on a downstream feedback signal, instead of directly inputting the first signal into a decoding apparatus for decoding, thereby ensuring long-term stability of signal encoding and decoding, avoiding deterioration or even failure, in long-term stability of signal encoding and decoding, that is caused due to signal non-stationarity, and meeting a service requirement. This application further provides a signal processing apparatus, a chip, a brain-computer interface system, a compute device cluster, a computer-readable storage medium, and a computer program product that correspond to the foregoing signal processing method.

According to a first aspect, this application provides a signal processing method. The method may be performed by a signal processing apparatus. The signal processing apparatus may be an apparatus that is in a brain-computer interface system and that is configured to process a signal acquired from a brain tissue. The signal processing apparatus may be a software apparatus, for example, software or a cloud service that is locally deployed. The software apparatus may be deployed in a compute device cluster (including at least one compute device), and the compute device cluster executes program code of the software apparatus, to perform the signal processing method in this application. The signal processing apparatus may alternatively be a hardware apparatus, for example, a compute device cluster having a signal processing function. When the hardware apparatus is run, the signal processing method in this application is performed.

Specifically, the signal processing apparatus may obtain a first signal, where the first signal is a signal acquired from the brain tissue, and obtain a downstream feedback signal. Then, the signal processing apparatus may process the first signal based on the feedback signal. In this way, the first signal can be processed based on the feedback signal, instead of directly inputting the first signal into a decoding apparatus for decoding, thereby ensuring long-term stability of signal encoding and decoding, avoiding deterioration or even failure, in long-term stability of signal encoding and decoding, that is caused due to signal non-stationarity, and meeting a requirement of a service (especially a service that is used for a long time, for example, a medical rehabilitation service).

In some possible implementations, the downstream feedback signal includes a feedback signal of the decoding apparatus or a feedback signal of an effector. Both the feedback signal of the decoding apparatus or a feedback signal of an effector controlled by the decoding apparatus may be used to determine decoding precision, and the signal is processed based on the decoding precision, for example, data integration is performed, to ensure decoding stability.

In some possible implementations, the first signal may be a signal acquired from the brain tissue at first time, the feedback signal represents a feedback on a second signal, the second signal is a signal acquired from the brain tissue at second time, and the second time is earlier than the first time. The first time and the second time may be moment or time periods (time sequences). In this method, a signal at current time may be processed by using a feedback on a signal at historical time, to ensure decoding stability.

In some possible implementations, the feedback signal includes a brain command or a brain status that is at the second time and that is obtained by decoding the second signal by the decoding apparatus, or the feedback signal includes an action command or an action status that is output by the effector in response to the brain command (brain command) or the brain status (brain status) that is at the second time. The brain command is a command sent by the brain tissue, and the command may usually be a control command, for example, a brain command for controlling movement of a prosthetic limb. The brain status is an activity status or an excitement status of at least one region of the brain tissue. The action command or the action status may be a command or a status output by the effector in response to the brain command or the brain status. When the effector is an external application (external application), the action command/action status may alternatively be an application command (application command) / application status (application status). For example, the application status may include a pose.

Decoding precision may be determined by using the brain command/brain status or the action command/action status, and signal processing is performed based on the decoding precision, for example, data integration is performed, to ensure decoding stability.

In some possible implementations, when determining, based on the feedback signal, that a trigger condition is met, the signal processing apparatus may process the first signal according to a processing policy corresponding to the trigger condition. The trigger condition may be a condition related to a monitoring indicator, and the monitoring indicator may be set based on a requirement. For example, the monitoring indicator may be the decoding precision. When the trigger condition is met, the signal processing apparatus processes the first signal according to the processing policy corresponding to the trigger condition, to ensure decoding stability. In addition, additional overheads that are caused due to signal processing when decoding is stable can be avoided.

In some possible implementations, the processing policy includes at least one of the following: calibration, coordination control, data integration, screening of a stable distribution channel, or paradigm update. When the trigger condition is met, the processing policy corresponding to the trigger condition is used for signal processing, so that targeted processing can be implemented, thereby improving decoding precision and decoding stability.

In some possible implementations, the feedback signal includes the brain command or the brain status. The signal processing apparatus may determine decoding precision based on the brain command or the brain status in the feedback signal and a target brain command or a target brain status, and then update, based on the decoding precision, a decoding parameter for decoding the first signal. In this way, signal calibration can be implemented, and decoding stability can be improved by performing signal calibration. It should be noted that, when the signal calibration is performed, regular calibration may be used, or adaptive calibration may be used. The adaptive calibration is triggered when the decoding precision is less than a specified value, so that unnecessary overheads can be reduced.

In some possible implementations, the feedback signal includes at least one of the brain command or the brain status and at least one of the action command or the action status. Correspondingly, the signal processing apparatus may determine a first motion intent of a user based on the brain command or the brain status, predict a second motion intent of the user based on the action command or the action status, determine a ratio of the first motion intent to the second motion intent, and send the ratio to the decoding apparatus.

In this way, coordination control can be implemented for a motion intent obtained through decoding and a motion intent predicted based on an algorithm, and decoding stability can be ensured. The coordination control may be classified into time-shared coordination control or adaptive coordination control. The adaptive coordination control supports human intervention in an algorithm control process, and features high reliability.

In some possible implementations, the feedback signal includes the brain command or the brain status. The signal processing apparatus may determine decoding precision based on the brain command or the brain status in the feedback signal and a target brain command or a target brain status. When the decoding precision meets a first specified condition, distribution stability of a plurality of acquisition channels is determined, and information about an acquisition channel whose distribution stability meets a requirement is sent to the decoding apparatus, to cause the decoding apparatus to decode the first signal based on the acquisition channel whose delivery stability meets the requirement.

The first specified condition may be that a decrease amplitude of the decoding precision within a specified time period is greater than a specified amplitude, indicating that there is a sudden sharp drop (also referred to as a sharp decrease) in the decoding precision. Considering that the sharp decrease in the decoding precision may be caused due to electrode deactivation or a change in a distribution characteristic, the signal processing apparatus determines the distribution stability of the plurality of acquisition channels, and sends, to the decoding apparatus, the information about the acquisition channel whose distribution stability meets the requirement, so as to improve decoding stability.

In some possible implementations, the feedback signal includes the brain command or the brain status. The signal processing apparatus may determine decoding precision based on the brain command or the brain status in the feedback signal and a target brain command or a target brain status. When the decoding precision meets a second specified condition, a task parameter is updated, and a first signal obtained through modulation based on an updated task parameter is sent to the decoding apparatus.

The second specified condition may be that the decoding precision remains lower than a precision threshold within a specified time period, indicating that the decoding precision remains low for a period of time. A reason why the decoding precision remains a low level may be that an existing task is excessively challenging/difficult for the user. Based on this, the signal processing apparatus may update the task parameter, and send, to the decoding apparatus, the first signal obtained through modulation based on an updated task parameter. In this way, a difficulty level of the task can be reduced, thereby ensuring decoding stability.

In some possible implementations, the feedback signal includes the brain command or the brain status. The signal processing apparatus may determine decoding precision based on the brain command or the brain status in the feedback signal and a target brain command or a target brain status. When the decoding precision meets a third specified condition, the first signal is used to perform fine-tuning on a pre-trained model, to obtain an updated decoding parameter, and the updated decoding parameter is sent to the decoding apparatus, where the pre-trained model is obtained by pre-training a decoding model in the decoding apparatus based on historical data.

The third specified condition may be that the decoding precision remains lower than a precision threshold within a specified time period, indicating that the decoding precision remains low for a period of time. The signal processing apparatus may consider loading the pre-trained model, and performing fine-tuning based on a small amount of data on a current day. Specifically, the signal processing apparatus may perform fine-tuning on the pre-trained model by using the first signal, to obtain an updated decoding parameter, and send the updated decoding parameter to the decoding apparatus. In this way, data integration can be performed, and decoding stability can be improved.

In some possible implementations, the first signal is a digital signal. The signal processing apparatus may obtain a neural signal acquired from the brain tissue, convert the neural signal into an analog signal, and convert the analog signal into the digital signal through analog-to-digital conversion. In this way, a foundation may be laid out for implementing brain-computer interaction.

In some possible implementations, the signal processing apparatus may preprocess the analog signal or the digital signal, to implement signal compression. The analog signal and the digital signals are preprocessed, and signal quality can be improved, and a signal data amount can be reduced.

In some possible implementations, the signal processing apparatus may preprocess the analog signal or the digital signal on a hardware signal acquisition apparatus side. In this way, when a brain-computer interface acquisition technology is developed toward high throughput, high precision, a high sampling rate, and wireless transmission, analog signal preprocessing and/or digital signal preprocessing can also be performed on a sensing end, and a function signal can be extracted from an original bioelectrical signal (an analog electrical signal converted from a neural signal), thereby reducing a communication bandwidth and power consumption pressure, and implementing integrated sensing and computing.

In some possible implementations, the neural signal includes a signal generated by the brain tissue by receiving a stimulus signal output by a stimulation apparatus, the stimulus signal is generated based on a result of encoding an external input signal that is of at least one modality and that is acquired by a prosthesis, and the encoding result includes a parameter of a stimulation mode matching the modality.

In this method, independent generation of a stimulus signal is also supported, to stimulate the brain tissue to generate a neural signal, so as to implement brain-computer interaction.

In some possible implementations, the prosthesis is a wearable device, for example, smart glasses or a smart head-mounted display. A sensor of at least one modality is deployed on the wearable device, and the wearable device is configured to acquire the external input signal of the at least one modality via the sensor of the at least one modality.

The stimulus signal is generated by using the result of encoding the external input signal, to stimulate a corresponding functional region of the brain tissue, so as to help a prosthesis user implement sensing, for example, help a blind person sense a location and a size of an obstacle, or sense a traffic light status, thereby improving convenience.

In some possible implementations, the neural signal includes a signal generated by the brain tissue by receiving a stimulus signal output by a stimulation apparatus, the stimulus signal is generated based on a result of encoding an internal input signal that is of at least one modality and that is generated when the decoding apparatus obtains a specified brain status or brain command through decoding, and the encoding result includes a parameter of a stimulation mode matching the modality. In this way, brain-computer interaction for which acquisition and stimulation are combined can be implemented, and a requirement of a corresponding service scenario can be met.

According to a second aspect, this application provides a signal processing apparatus. The apparatus includes:
an obtaining unit, configured to: obtain a first signal, where the first signal is a signal acquired from a brain tissue, and obtain a downstream feedback signal; and
a processing unit, configured to process the first signal based on the feedback signal.

In some possible implementations, the downstream feedback signal includes a feedback signal of a decoding apparatus or a feedback signal of an effector.

In some possible implementations, the first signal is a signal acquired from the brain tissue at first time, the feedback signal represents a feedback on a second signal, the second signal is a signal acquired from the brain tissue at second time, and the second time is earlier than the first time.

In some possible implementations, the feedback signal includes a brain command or a brain status that is at the second time and that is obtained by decoding the second signal by the decoding apparatus, or the feedback signal includes an action command or an action status that is output by the effector in response to the brain command or the brain status that is at the second time.

In a possible implementation, the processing unit is specifically configured to:
process, when determining, based on the feedback signal, that a trigger condition is met, the first signal according to a processing policy corresponding to the trigger condition.

In some possible implementations, the processing policy includes at least one of the following:
calibration, coordination control, data integration, screening of a stable distribution channel, or paradigm update.

In some possible implementations, the feedback signal includes a brain command or a brain status, and the processing unit is specifically configured to:
determine decoding precision based on the brain command or the brain status in the feedback signal and a target brain command or a target brain status; and
update, based on the decoding precision, a decoding parameter for decoding the first signal.

In some possible implementations, the feedback signal includes at least one of a brain command or a brain status and at least one of an action command or an action status, and the processing unit is specifically configured to:
determine a first motion intent of a user based on the brain command or the brain status, and predict a second motion intent of the user based on the action command or the action status; and
determine a ratio of the first motion intent to the second motion intent, and send the ratio to the decoding apparatus.

In some possible implementations, the feedback signal includes the brain command or the brain status that is at the second time, and the processing unit is specifically configured to:
determine decoding precision based on the brain command or the brain status in the feedback signal and a target brain command or a target brain status; and
when the decoding precision meets a first specified condition, determine distribution stability of a plurality of acquisition channels, and send, to the decoding apparatus, information about an acquisition channel whose distribution stability meets a requirement, to cause the decoding apparatus to decode the first signal based on the acquisition channel whose distribution stability meets the requirement.

In some possible implementations, the feedback signal includes a brain command or a brain status, and the processing unit is specifically configured to:
determine decoding precision based on the brain command or the brain status in the feedback signal and a target brain command or a target brain status; and
when the decoding precision meets a second specified condition, update a task parameter, and send, to the decoding apparatus, a first signal obtained through modulation based on an updated task parameter.

In some possible implementations, the feedback signal includes a brain command or a brain status, and the processing unit is specifically configured to:
determine decoding precision based on the brain command or the brain status in the feedback signal and a target brain command or a target brain status; and
when the decoding precision meets a third specified condition, perform fine-tuning on a pre-trained model by using the first signal, to obtain an updated decoding parameter, and send the updated decoding parameter to the decoding apparatus, where the pre-trained model is obtained by pre-training a decoding model in the decoding apparatus based on historical data.

In some possible implementations, the first signal is a digital signal, and the obtaining unit is specifically configured to:
obtain a neural signal acquired from the brain tissue;
convert the neural signal into an analog signal; and
convert the analog signal into the digital signal through analog-to-digital conversion.

In some possible implementations, the processing unit is further configured to:
preprocess the analog signal or the digital signal via a signal preprocessing module, to implement signal compression.

In some possible implementations, the signal preprocessing module is integrated into or deployed on a hardware signal acquisition apparatus, and the processing unit is specifically configured to:
preprocess the analog signal or the digital signal on the hardware signal acquisition apparatus side via the signal preprocessing module.

In some possible implementations, the neural signal includes a signal generated by the brain tissue by receiving a stimulus signal output by a stimulation apparatus, the stimulus signal is generated based on a result of encoding an external input signal that is of at least one modality and that is acquired by a prosthesis, and the encoding result includes a parameter of a stimulation mode matching the modality.

In some possible implementations, the prosthesis is a wearable device, a sensor of at least one modality is deployed on the wearable device, and the wearable device is configured to acquire the external input signal of the at least one modality via the sensor of the at least one modality.

In some possible implementations, the neural signal includes a signal generated by the brain tissue by receiving a stimulus signal output by a stimulation apparatus, the stimulus signal is generated based on a result of encoding an internal input signal that is of at least one modality and that is generated when the decoding apparatus obtains a specified brain status or brain command through decoding, and the encoding result includes a parameter of a stimulation mode matching the modality.

According to a third aspect, this application provides a chip. The chip includes a processor and a communication interface. The communication interface is configured to obtain a first signal, the first signal is a signal acquired from a brain tissue, and the processor is configured to execute computer-readable instructions, to perform the signal processing method according to any one of the first aspect or the implementations of the first aspect.

According to a fourth aspect, this application provides a brain-computer interface system. The system includes a signal processing apparatus and a hardware signal acquisition apparatus. The signal processing apparatus is configured to perform the signal processing method according to any one of the first aspect or the implementations of the first aspect, to process a signal output by the hardware signal acquisition apparatus, and implement brain-computer interaction.

In some possible implementations, the signal processing apparatus includes a signal preprocessing module, where the signal preprocessing module is integrated into the hardware signal acquisition apparatus for deployment, and the signal preprocessing module is configured to:
preprocess, on the hardware signal acquisition apparatus side, the signal output by the hardware signal acquisition apparatus.

In some possible implementations, the system further includes:
a stimulation apparatus, configured to generate a stimulus signal based on a result of encoding an external input signal that is of at least one modality and that is acquired by a prosthesis, where the encoding result includes a parameter of a stimulation mode matching the modality, and the stimulus signal is used to stimulate a brain tissue.

In some possible implementations, the system further includes:
a stimulation apparatus, configured to generate a stimulus signal based on a result of encoding an internal input signal that is of at least one modality and that is generated when a decoding apparatus obtains a specified brain status or brain command through decoding, where the encoding result includes a parameter of a stimulation mode matching the modality, and the stimulus signal is used to stimulate a brain tissue.

According to a fifth aspect, this application provides a compute device cluster. The compute device cluster includes at least one compute device, and the at least one compute device includes at least one processor and at least one memory. The at least one processor and the at least one storage communicate with each other. The at least one processor is configured to execute instructions stored in the at least one memory, to cause a compute device or the compute device cluster to perform the signal processing method according to any one of the first aspect or the implementations of the first aspect.

According to a sixth aspect, this application provides a computer-readable storage medium. The computer-readable storage medium stores instructions, and the instructions instruct a compute device or a compute device cluster to perform the signal processing method according to any one of the first aspect or the implementations of the first aspect.

According to a seventh aspect, this application provides a computer program product including instructions. When the computer program product runs on a compute device or a compute device cluster, the compute device or the compute device cluster is caused to perform the signal processing method according to any one of the first aspect or the implementations of the first aspect.

In this application, based on the implementations provided in the foregoing aspects, the implementations may be further combined, to provide more implementations.

### BRIEF DESCRIPTION OF DRAWINGS

To describe the technical methods in embodiments of this application more clearly, the following briefly describes the accompanying drawings for describing embodiments.
FIG. 1 is a diagram of an architecture of a brain-computer interface system according to an embodiment of this application;
FIG. 2 is a diagram of an architecture of another brain-computer interface system according to an embodiment of this application;
FIG. 3 is a diagram of an architecture of still another brain-computer interface system according to an embodiment of this application;
FIG. 4 is a diagram of a signal preprocessing procedure according to an embodiment of this application;
FIG. 5 is a hierarchical logic diagram of a storage system according to an embodiment of this application;
FIG. 6 is a diagram of different brain-computer interaction modes according to an embodiment of this application;
FIG. 7 is a diagram of a scenario of a signal processing method according to an embodiment of this application;
FIG. 8 is a diagram of a scenario of a signal processing method according to an embodiment of this application;
FIG. 9 is a diagram of a scenario of a signal processing method according to an embodiment of this application;
FIG. 10 is a diagram of a scenario of a signal processing method according to an embodiment of this application;
FIG. 11 is a diagram of a scenario of a signal processing method according to an embodiment of this application;
FIG. 12 is a diagram of a scenario of another signal processing method according to an embodiment of this application;
FIG. 13 is a diagram of a scenario of another signal processing method according to an embodiment of this application;
FIG. 14 is a diagram of a structure of a signal processing apparatus according to an embodiment of this application;
FIG. 15 is a diagram of a structure of a compute device according to an embodiment of this application; and
FIG. 16 is a diagram of a structure of a compute device cluster according to an embodiment of this application.

### DESCRIPTION OF EMBODIMENTS

The terms "first" and "second" in embodiments of this application are merely intended for description, and shall not be understood as an indication or implication of relative importance or an implicit indication of a quantity of indicated technical features. Therefore, a feature limited by the term "first" or "second" may explicitly or implicitly include one or more features.

First, some technical terms in embodiments of this application are described.

A brain-computer interface (brain-computer interface, BCI) shows the intensive development of a human-computer interface (Human-Computer Interface, HCI) technology. A conventional human-computer interaction technology needs to be implemented by using software and hardware at a medium layer, for example, a computer display, a visualized interface, and a mouse. The brain-computer interface is a new human-computer interaction mode based on neuroscience and engineering technologies, and may establish, by obtaining and analyzing a brain signal in real time, a channel for direct communication and control between a brain tissue (for example, a human brain and an animal brain) and a computer or another electronic device, to directly convert an activity of a central nervous system into information for output, so as to understand brain consciousness. In addition, bioengineering means such as optogenetic means and a brain electrode may be used to intervene in or even participate in a brain activity, and a user can express an idea or manipulate an external device without using a language or body movement.

Brain-computer interface technologies are classified, based on implantation degrees of a hardware signal acquisition apparatus (which may also be referred to as a hardware signal acquisition (hardware signal acquisition) module), into two types: an intrusive brain-computer interface technology and a non-intrusive brain-computer interface technology.

A non-intrusive brain-computer interface means that the hardware signal acquisition apparatus is disposed on a scalp and a region outside the scalp, and the hardware signal acquisition apparatus can be worn outside a body. The non-intrusive brain-computer interface mainly acquires an electroencephalogram (Electroencephalogram, EEG) signal of the scalp, and the EEG is an electrical signal formed by a plurality of local field potentials passing through a cortex. The non-intrusive brain-computer interface may be applied to scenarios, for example, evaluation and control of a physiological status (detection of attention, sleep, and pressure), assisted human-machine interaction (disability assistance, and augmented reality/virtual reality), and fitness and health.

An intrusive brain-computer interface means that the hardware signal acquisition apparatus is disposed inside the scalp, and usually can be implanted through surgery. The invasive brain-computer interface mainly acquires a spike (spike) signal, a local field potential (Local field potential, LFP), and an electrocorticography (Electrocorticography, ECoG). The local field potential represents a sum of electrical signals of neurons in a local region of an electrode. The electrocorticography is similar to the local field potential, but can record only an electrical signal on a surface of the cortex. The intrusive brain-computer interface acquires a high-quality signal and may be used in a medical rehabilitation scenario.

The following provides example descriptions of a brain-computer interface system with reference to the foregoing explanations of concepts related to the brain-computer interface. The brain-computer interface system is a system for processing a signal acquired from the brain tissue, and therefore may also be referred to as a signal processing system or an electroencephalogram signal processing system.

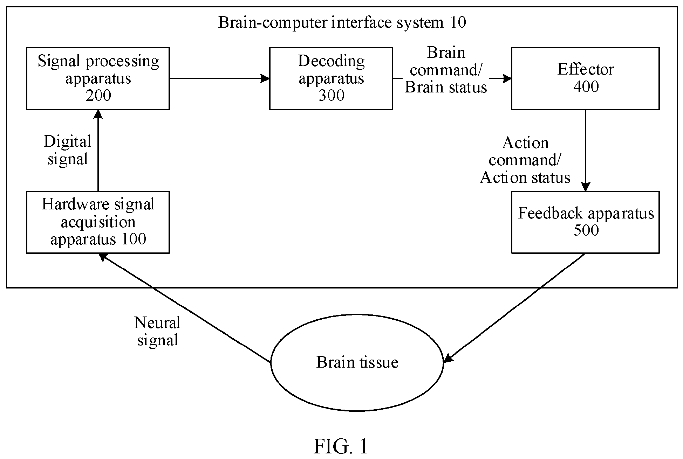

As shown in FIG. 1, a brain-computer interface system 10 includes a hardware signal acquisition (hardware signal acquisition) apparatus 100 (also referred to as a hardware signal acquisition module), a signal processing (signal processing) apparatus 200 (or referred to as a signal processing module), a decoding (decoding) apparatus 300 (or referred to as a decoding module), an effector (effector) 400, and a feedback (feedback) apparatus 500 (or referred to as a feedback module). After performing signal processing, the signal processing apparatus 200 may further store a processed signal, to facilitate subsequent use. Based on this, the signal processing apparatus 200 may be a signal processing and storage apparatus. Similarly, the decoding apparatus 300 is configured to: perform feature extraction (feature extraction) on the processed signal, and then decode an extracted feature. Therefore, the decoding apparatus 300 may be a feature extraction and decoding apparatus.

The hardware signal acquisition apparatus 100 is connected to the signal processing apparatus 200, the signal processing apparatus 200 is connected to the decoding apparatus 300, and the decoding apparatus 300 is connected to the effector 400. The effector 400 may directly act on a brain tissue, or may be connected to the feedback apparatus 500, and act on a brain tissue via the feedback apparatus 500.

The following describes in detail functions, inputs, outputs, and the like of the hardware signal acquisition apparatus 100, the signal processing apparatus 200, the decoding apparatus 300, the effector 400, and the feedback apparatus 500.

The hardware signal acquisition apparatus 100 is configured to acquire a signal from the brain tissue. The hardware signal acquisition apparatus 100 may be disposed on a scalp and a region outside the scalp, to acquire the signal in a non-intrusive manner. The hardware signal acquisition apparatus 100 may alternatively be implanted into the scalp, to acquire the signal in an intrusive manner. As shown in Table 1, the input of the hardware signal acquisition apparatus 100 may be a neural signal (neural signal) of the brain tissue, and the output of the hardware signal acquisition apparatus 100 may be a digital signal. The hardware signal acquisition apparatus 100 uses an electrode, a circuit, and the like to convert the neural signal into an electrical signal, for example, an analog electrical signal (also referred to as an analog signal), and convert the electrical signal into a processable digital electrical signal (also referred to as a digital signal). It should be noted that the analog signal and the digital signal may be collectively referred to as an electroencephalogram signal.

**Table 1**

| Apparatus (Module) | Input | Output | Function |
|---|---|---|---|
| Hardware signal acquisition apparatus 100 | Neural signal | Digital signal | Converts the neural signal into an electrical signal via an electrode, a circuit, and the like, and converts the electrical signal into the processable digital signal |
| Signal processing apparatus 200 | Digital signal | Processed digital signal | Improves signal quality, and further supports long-term storage for subsequent processing |
| Decoding apparatus 300 | Processed digital signal | Brain command/ Brain status | Extracts or translates the meaningful brain command or brain status from the processed digital signal |
| Effector 400 | Brain command/ Brain status | Action command/Action status | Receives the extracted brain command or brain status, and translates the brain command or brain status into the action command or action status of the effector |
| Feedback apparatus 500 | Action command/ Action status | Feedback on an execution result | Provides a system feedback, and establishes a closed loop between a brain action/brain status of a user and a system response |

The signal processing apparatus 200 is configured to perform signal processing on the digital signal output by the hardware signal acquisition apparatus 100, to improve signal quality. The signal processing apparatus 200 further has a storage function, and is configured to store the processed digital signal, to facilitate subsequent use. The input of the signal processing apparatus 200 may be the digital signal output by the signal acquisition apparatus 100, and the output of the signal processing apparatus 200 may be the processed digital signal, which is usually a high-quality digital signal.

The decoding apparatus 300 is configured to decode the processed signal from the signal processing apparatus 200, to obtain the brain command or brain status, so as to implement brain consciousness sensing. The input of the decoding apparatus 300 may be the processed digital signal output by the signal processing apparatus 200, and the output of the decoding apparatus may be the brain command or brain status. The brain command is a command sent by the brain tissue, and the command may usually be a control command, for example, a brain command for controlling movement of a prosthetic limb. The brain status is an activity status or an excitement status of at least one region of the brain tissue. Different regions of the brain tissue may correspond to different functions. For example, the cerebellum is responsible for regulating and controlling a refined action, a posture, and balance, and the cerebral cortex is usually related to a high-level brain function, for example, conscious thought and action selection and control. During specific implementation, the decoding apparatus 300 may extract a feature from the processed signal, and then perform decoding based on the feature, to obtain the brain command or the brain status. It should be noted that features extracted by the decoding apparatus 300 may be different based on different signal types.

The effector 400 is configured to perform, in response to the brain command or the brain status, an action corresponding to the brain command or the brain status. As an object controlled by the decoding apparatus 300, the effector 400 may usually be at least one of an external device or a stimulation device. In some cases, the external device is also referred to as a peripheral or an external application (external application), and includes but is not limited to a computer cursor, a manipulator (mechanical arm), a mechanical skeleton, a wheelchair, and a monitoring and security protection device. The stimulation device may also be referred to as a stimulation apparatus, and may include the following types: a muscle stimulation device, and a neuro-modulation device. The muscle stimulation device may be a functional electrical stimulation (Functional Electrical Stimulation, FES) for a muscle. For the FES, a low-frequency spike current can be applied or amplified through signal-to-current conversion and then sent to a human body to produce an immediate effect, artificially causing the human body that is paralyzed from an injury to a central nervous system to make an action. The neuro-modulation device may include but is not limited to deep brain stimulation (Deep Brain Stimulation, DBS), spinal cord stimulation (spinal cord stimulation, SCS), and peripheral nerve stimulation (peripheral nerve stimulation, PNS). The input of the effector 400 may be the brain command or brain status, and the output of the effector 400 may be the action command or action status.

The feedback apparatus 500 is configured to: provide the system feedback for the brain tissue, and establish the closed loop between the brain action/brain status of the user and the system response. The input of the feedback apparatus 500 may be the action command or the action status output by the effector 400, and the output of the feedback apparatus 500 may be the feedback on the execution result (that is, the system feedback).

It should be noted that the feedback apparatus 500 is an optional apparatus. For example, the effector 400 may alternatively directly provide a feedback, in a form of a visual feedback, for the brain tissue, without using the feedback apparatus 500. An example in which the effector 400 is the computer cursor is used for description. A movement of the computer cursor may be fed back to the brain tissue in the form of visual feedback. In some cases, the feedback apparatus 500 may be used as a supplement to the visual feedback. For example, with a mechanical arm controlled by a brain-computer interface, a quadriplegic may usually complete a functional motion. However, a feedback provided by the vision to the quadriplegic is limited, while a gripping-type motion causes signal transfer in a form of a haptic feedback via the feedback apparatus 500, which is usually faster, more accurate, and more realistic.

However, a signal acquired by the intrusive brain-computer interface or the non-intrusive brain-computer interface is greatly affected by factors such as electrode deactivation, a random noise, a motion artifact (an interference signal caused due to a slight movement, for example, shaking of the head, of a participant in a brain-computer interaction process), and a physical and mental status, and is not stationary. Consequently, deterioration or even failure in long-term stability of signal encoding and decoding is caused, and a service requirement can hardly be met.

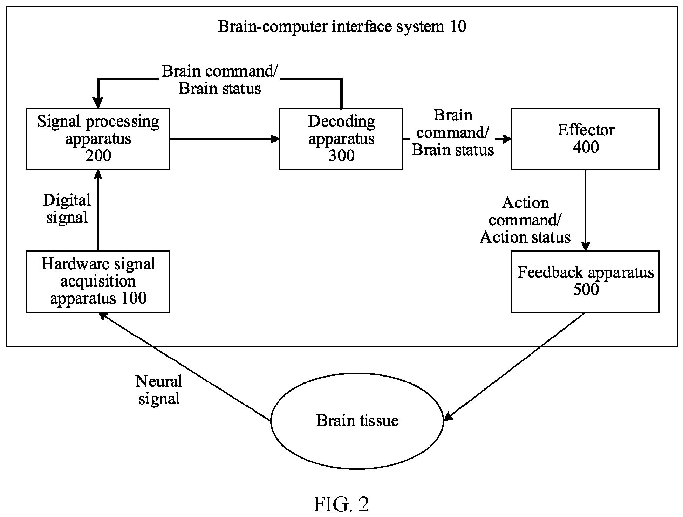

In view of this, this application provides a signal processing method. The method may be applied to a brain-computer interface system 10 shown in FIG. 2. Different from arrangement in the brain-computer interface system 10 shown in FIG. 1, in the brain-computer interface system 10 shown in FIG. 2, a feedback path to a signal processing apparatus 200 is disposed downstream in the signal processing apparatus 200. The feedback path may be a feedback path between a downstream effector 400 and the signal processing apparatus 200, or a feedback path between a downstream decoding apparatus 300 and the signal processing apparatus 200. The feedback path is used for transmitting a feedback signal of the effector 400 or the decoding apparatus 300 to the signal processing apparatus 200. Correspondingly, the signal processing apparatus 200 may process, based on the feedback signal, a signal (also referred to as a first signal) acquired from a brain tissue, instead of directly inputting the signal into the decoding apparatus 300 for decoding, thereby ensuring long-term stability of signal encoding and decoding, avoiding deterioration or even failure, in long-term stability of signal encoding and decoding, that is caused due to signal non-stationarity, and meeting a service requirement.

To make the technical solutions of this application clearer and easier to understand, the following describes, with reference to the accompanying drawings, a system architecture of the brain-computer interface system 10 provided in this application.

Refer to a diagram of an architecture of the brain-computer interface system 10 in FIG. 2. The brain-computer interface system 10 includes a hardware signal acquisition apparatus 100, a signal processing apparatus 200, a decoding apparatus 300, an effector 400, and a feedback apparatus 500. The hardware signal acquisition apparatus 100 is connected to the signal processing apparatus 200, the signal processing apparatus 200 is connected to the decoding apparatus 300, and the decoding apparatus 300 is connected to the effector 400. The effector 400 may directly act on a brain tissue, or may be connected to the feedback apparatus 500, and act on a brain tissue via the feedback apparatus 500. In addition, the feedback path to the signal processing apparatus 200 is further disposed downstream in the signal processing apparatus 200. In FIG. 2, an example in which the feedback path is disposed between the decoding apparatus 300 and the signal processing apparatus 200 is used for description. The feedback path is configured to provide the feedback signal for the signal processing apparatus 200, to assist the signal processing apparatus 200 in performing signal processing.

The following describes, with reference to Table 2, functions, inputs, and outputs of the apparatuses/modules in the brain-computer interface system 10 shown in FIG. 2.

**Table 2**

| Apparatus (Module) | Input | Output | Function |
|---|---|---|---|
| Hardware signal acquisition apparatus 100 | Neural signal | Digital signal | Converts the neural signal into an electrical signal via an electrode, a circuit, and the like, and converts the electrical signal into the processable digital signal |
| Signal processing apparatus 200 | 1. Digital signal | Processed digital signal | 1. Improves signal quality, and further supports long-term storage for subsequent processing |
| | 2. Brain command/Brain status | | |
| | | | 2. Performs signal processing based on a feedback signal, to improve decoding stability and accuracy |
| Decoding apparatus 300 | Processed digital signal | 1. Brain command/Brain status (to the effector 400) | Extracts or translates the meaningful brain command or brain status from the processed digital signal |
| | | 2. Brain command/Brain status (to the signal processing apparatus 200) | |
| Effector 400 | Brain command/ Brain status | Action command/ Action status | Receives the extracted brain command or brain status, and translates the brain command or brain status into the action command or action status of the effector |
| Feedback apparatus 500 | Action command/ Action status | Feedback on an execution result | Provides a system feedback, and establishes a closed loop between a brain action/brain status of a user and a system response |

For functions, inputs, and outputs of the hardware signal acquisition apparatus 100, the effector 400, and the feedback apparatus 500, refer to descriptions of the related content in Table 1 or FIG. 1. On the basis of Table 1, the feedback signal of the decoding apparatus 300 is further added to the input of the signal processing apparatus 200, and the feedback signal may be the brain command or the brain status. Correspondingly, the signal processing apparatus 200 is configured to process the first signal based on the feedback signal, to improve decoding stability. The first signal may be a signal acquired from the brain tissue at first time (for example, at current time or at a current moment), a second signal may be a signal acquired from the brain tissue at second time (for example, at historical time or at a historical moment), and the feedback signal represents a feedback of the decoding apparatus 300 on the second signal. In other words, the signal processing apparatus 200 may process a signal at the current time with reference to a brain command or a brain status at the historical time (for example, at a previous moment), which includes selecting/adjusting a signal post-processing (post-processing) procedure, to improve decoding stability. On the basis of Table 1, the output to the signal processing apparatus 200 is added to the output of the decoding apparatus 300. Specifically, the brain command/brain status is output to the signal processing apparatus 200, to cause the signal processing apparatus 200 to perform signal processing based on a feedback signal, for example, the brain command/brain status.

When the feedback path is disposed between the effector 400 and the signal processing apparatus 200, the feedback signal may be an action command or an action status. The signal processing apparatus 200 may process the signal with reference to an action command or an action status at a previous moment, which includes selecting/adjusting a signal post-processing (post-processing) procedure, to improve decoding stability. On the basis of Table 1, the output to the signal processing apparatus 200 is added to the output of the effector 400. Specifically, an action command/action status is output to the signal processing apparatus 200, to cause the signal processing apparatus 200 to perform signal processing based on a feedback signal, for example, the action command/action status.

The signal processing apparatus 200 is further configured to store a trigger condition and a processing policy corresponding to the trigger condition. Correspondingly, the signal processing apparatus 200 is configured to: process when determining, based on the feedback signal, that the trigger condition is met, the first signal according to the processing policy corresponding to the trigger condition.

The trigger condition may be a condition related to a monitoring indicator. The monitoring indicator may be set based on a service requirement. For example, the monitoring indicator may include decoding precision. The decoding precision may be determined based on a brain command or a brain status that is at the second time and that is obtained through decoding and a target brain command or a target brain status.

The processing policy may include at least one of calibration, coordination control, data integration, screening of a stable distribution channel, or paradigm update. The calibration may include regular calibration and adaptive calibration. Similar to the calibration, the coordination control may also include time-shared coordination control or adaptive coordination control. Specific implementations of calibration, coordination control, data integration, screening of a stable distribution channel, or paradigm update are described in detail below.

In the system shown in FIG. 2, the first signal processed by the signal processing apparatus 200 may be a digital signal. The digital signal may be obtained by the hardware signal acquisition apparatus 100 through acquisition and processing. Specifically, the hardware signal acquisition apparatus 100 is configured to: acquire a neural signal from the brain tissue, convert the neural signal into a model signal, and then convert the analog signal into the digital signal through analog-to-digital conversion, for example, via an analog-to-digital converter (Analog-to-Digital Converter, ADC). The signal processing apparatus 200 obtains the digital signal from the hardware signal acquisition apparatus 100, and performs signal processing.

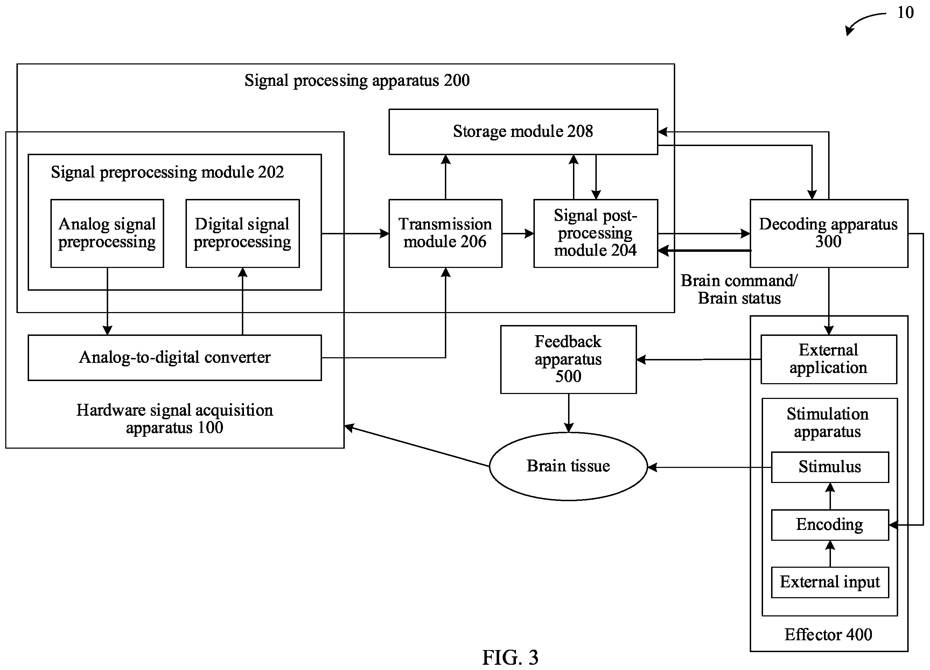

In some possible implementations, refer to FIG. 3. The signal processing apparatus 200 may include a signal preprocessing (signal preprocessing) module 202 and a signal post-processing (signal post-processing) module 204. The signal preprocessing module 202 and the signal post-processing module 206 may perform signal transmission via a transmission (transmission) module 206. The transmission module 206 may be a module that implements signal transmission based on a wired or wireless technology. The wireless technology may include but is not limited to Bluetooth or a wireless network (for example, Wi-Fi).

The signal preprocessing module 202 is configured to preprocess the signal acquired by the hardware signal acquisition apparatus 100. Preprocessing may include at least one of analog signal preprocessing or digital signal preprocessing. The signal preprocessing module 202 may be integrated into or deployed on the hardware signal acquisition apparatus 100, to preprocess the signal at a sensing end (for example, the hardware signal acquisition apparatus 100), to implement signal compression.

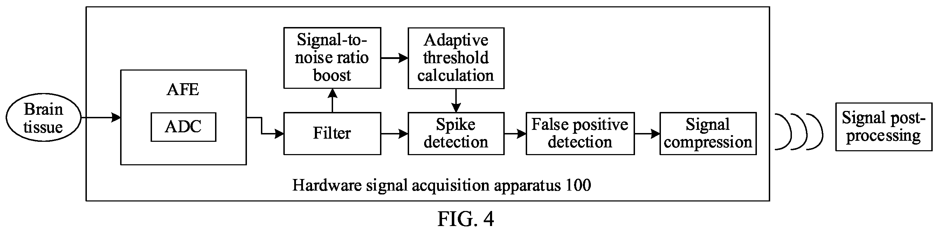

As shown in FIG. 4, a neural signal acquired in a brain tissue may be processed and converted, via an analog front-end (Analog Front-end) chip in a hardware signal acquisition apparatus 100, into an electrical signal that can be processed by a compute device. An ADC may be integrated into the AFE. Therefore, the electrical signal that can be processed and that is obtained by the AFE through conversion may be a digital signal. The digital signal may be preprocessed by at least one module in a signal preprocessing module 202: a filtering (filter) module, a signal-to-noise ratio boost (SNR Boost) module, an adaptive threshold (Adaptive Threshold) calculation module, a spike detection (Spike Detection) module, a false positive detection (false positive reduction, FP Reduction) module, or a signal compression (for example, spike compression Spike Compression) module. The false positive detection means detecting a noise that is identified as a wave crest, and the false positive detection may be implemented through waveform detection. The signal preprocessing module 202 may be integrated into or deployed on the hardware signal acquisition apparatus 100 in an on-chip (on-chip implementation) manner. An input of the signal preprocessing module 202 may be a voltage signal obtained by the ADC through conversion, and an output of the signal preprocessing module 202 may include but is not limited to modalities such as a spike waveform, a time point for firing a spike, a spike firing frequency in a specific time window, and a specific spectrum signal.

A preprocessed digital signal may be sent to a transmission module 206 according to a corresponding transmission protocol, and the transmission module 206 transmits the preprocessed digital signal to a signal post-processing module 204 according to the corresponding transmission protocol. It should be noted that FIG. 4 merely describes an example of a preprocessing process of a digital signal. An analog signal may also be preprocessed. For example, the analog signal may enter an amplifier or a filter for preprocessing.

In this way, when a brain-computer interface acquisition technology is developed toward high throughput, high precision, a high sampling rate, and wireless transmission, analog signal preprocessing and/or digital signal preprocessing can also be performed on a sensing end, and a function signal can be extracted from an original bioelectrical signal (an analog electrical signal converted from a neural signal), thereby reducing a communication bandwidth and power consumption pressure, and implementing integrated sensing and computing.

In some possible implementations, the signal processing apparatus 200 may further include a storage module 208, which is also referred to as a storage system (storage system). The preprocessed signal may be transmitted to the storage module 208 via the transmission module 206 for storage, to facilitate subsequent use. It should be noted that the digital signal obtained by the ADC through conversion may alternatively be directly sent to the transmission module 206 without being preprocessed. The transmission module 206 may transmit the digital signal to the signal post-processing module 204, and the transmission module 206 may alternatively transmit the digital signal to the storage module 208, to facilitate subsequent processing. For example, the signal post-processing module 204 may read the stored digital signal from the storage module 208, to perform signal processing (for example, post-processing).

The signal post-processing module 204 is configured to process the first signal based on a feedback signal (for example, a brain command or a brain status fed back by the decoding apparatus 300, or an action command or an action status fed back by the effector 400, where the feedback signal of the decoding apparatus 300 is used as an example for description in FIG. 3). As shown in FIG. 3, a feedback path is disposed between the decoding apparatus 300 and the signal post-processing module 204 of the signal processing apparatus 200, and the feedback path is used to transmit the feedback signal of the decoding apparatus 300 to the signal post-processing module 204 of the signal processing apparatus 200. Correspondingly, the signal post-processing module 204 may process the first signal based on the brain command or the brain status (for example, visual information or pose information) fed back by the decoding apparatus 300. For example, regular or adaptive calibration, time-shared or adaptive coordination control, data integration, screening of a stable distribution channel, or paradigm optimization is performed, to improve long-term stability of decoding.

An example in which a signal is acquired from the brain tissue, and signal processing and decoding are performed on the signal, to control the effector 400 is used for the foregoing descriptions. In some possible implementations, the effector 400 may include a stimulation apparatus. The stimulation apparatus may generate a stimulus signal, to stimulate the brain tissue to generate a neural signal.

In some possible implementations, the stimulation apparatus may independently generate a stimulus signal. Specifically, the stimulation apparatus may obtain an external input signal that is of at least one modality and that is acquired by a prosthesis (for example, a visual prosthesis or an auditory prosthesis), and obtain an encoding result by encoding the external input signal of the at least one modality, where the encoding result includes a parameter of a stimulation mode matching the modality. The stimulation apparatus may generate a stimulus signal based on the encoding result.

The prosthesis may be a wearable device, including but not limited to smart glasses and a smart head-mounted display device. A sensor of at least one modality may be deployed on the wearable device, and the wearable device is configured to acquire the external input signal of the at least one modality via the sensor of the at least one modality. An example in which the prosthesis is the smart glasses is used for description. The smart glasses may be deployed with an image sensor (for example, a camera). The image sensor may acquire an environment image, and recognize the environment image by using an image recognition model, to obtain obstacle information or traffic light status information. The stimulation apparatus may obtain the obstacle information or the traffic light status information, and encode the obstacle information or the traffic light status information to obtain an encoding result. Then, the stimulation apparatus generates a stimulus signal based on the encoding result, to stimulate a region responsible for vision in the brain tissue, so that a blind person wearing the smart glasses can sense a location and a size of an obstacle or sense a traffic light status.

In some other possible implementations, the stimulation apparatus may generate a stimulus signal based on acquired information. Specifically, when obtaining a specified brain status or brain command through decoding, the decoding apparatus may generate an internal input signal of at least one modality. For example, the brain status may be a state in which a user is about to experience epilepsy, and the decoding apparatus may generate an internal input signal for inhibiting the epilepsy. Correspondingly, the stimulation apparatus may obtain the internal input signal that is of the at least one modality and that is output by the decoding apparatus, and encode the internal input signal of the at least one modality to obtain an encoding result, where the encoding result includes a parameter of a stimulation mode matching the modality. The stimulation apparatus may generate a stimulus signal based on the encoding result. The stimulus signal may stimulate a region responsible for motion in the brain tissue, to inhibit the epilepsy for the user.

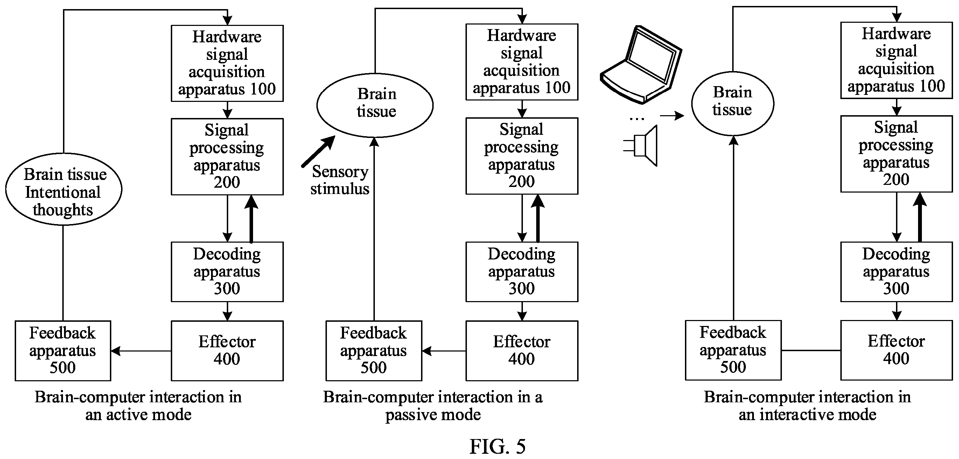

It should be noted that the brain-computer interface system 10 may implement brain-computer interaction in different modes. As shown in FIG. 5, a brain-computer interface system 10 may implement brain-computer interaction in an active mode, a passive mode, or an interactive mode. In the active mode, the brain-computer interface system 10 may control a peripheral (for example, a mechanical arm) through intentional thoughts (intentional thoughts). In the passive mode, when a user receives a specific sensory stimulus, the brain-computer interface system 10 may acquire a corresponding electroencephalogram signal (for example, a first signal), to control a peripheral. In the interaction mode, the brain-computer interface system 10 may trigger, based on occurrence of a specific perception, cognition, or motion event of an external environment, a related electroencephalogram signal to control a peripheral. Regardless of the active mode, the passive mode, or the interactive mode, a signal processing apparatus 200 in the brain-computer interface system 10 may receive a feedback signal, for example, a feedback signal (a brain command or a brain status) of a decoding apparatus 300, to determine decoding precision, so as to determine whether decoding is accurate or whether decoding is stable. When decoding is inaccurate or decoding is unstable, for example, a decrease amplitude of decoding precision/stability exceeds a specified threshold, a signal post-processing module 204 of the signal processing apparatus 200 may be triggered to perform post-processing on the first signal, to improve the decoding precision or decoding stability.

Based on the foregoing brain-computer interface system 10, this application provides a signal processing method. The following describes the signal processing method in this application with reference to embodiments.

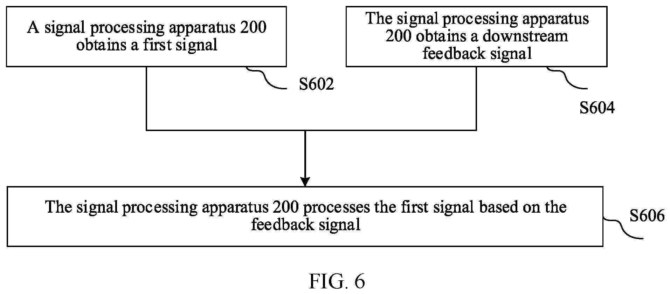

Refer to a flowchart of a signal processing method in FIG. 6. The method may be performed by the signal processing apparatus 200 in the brain-computer interface system 10, and the method includes the following steps.

S602: The signal processing apparatus 200 obtains a first signal.

The first signal is a signal acquired from a brain tissue. The first signal may be a digital signal. For example, the first signal may be a neural signal acquired from the brain tissue by the hardware signal acquisition apparatus 100. The neural signal may be converted into an analog signal, and then converted into a processable digital signal via an ADC.

In some possible implementations, the first signal may alternatively be a preprocessed signal, and signal quality may be improved through preprocessing. The preprocessing may include analog signal preprocessing and/or digital signal preprocessing. The analog signal preprocessing may include at least one of amplification and filtering (analog filtering). The digital signal preprocessing may include at least one of filtering (digital filtering), signal-to-noise ratio boost, adaptive threshold calculation, spike detection, waveform detection (for example, FP Reduction), and signal compression (for example, Spike Compression).

For a specific implementation of the digital signal preprocessing, refer to FIG. 4. A digital signal output by the AFE via an AFC may be filtered by a filter, and signal-to-noise ratio boost is performed, to improve a signal-to-noise ratio. When the adaptive threshold calculation needs to be performed, filtered signals (or converted raw data of the ADC if filtering is not performed) can be divided into two channels of signals. One channel of signals enter a spike detection module, and the other channel of signals enter an adaptive threshold calculation module. A threshold (threshold) is determined based on the two channels of outputs. After the threshold is determined, spike detection may be performed based on the threshold. Waveform detection or spike compression may be performed on a spike signal. In this way, a compression ratio as high as possible can be obtained when high precision and low power consumption are ensured.

It should be noted that the foregoing preprocessing process may be performed on a side of the hardware signal acquisition apparatus 100. As shown in FIG. 3, the signal processing apparatus 200 may include the signal preprocessing module 202 and the signal post-processing module 204. The signal preprocessing module 202 may be integrated into or deployed on the hardware signal acquisition apparatus 100. The signal processing apparatus 200 may preprocess a signal at a sensing end (for example, the hardware signal acquisition apparatus 100) via the signal preprocessing module 202, to implement signal compression. Even if the hardware signal acquisition apparatus 100 acquires a signal based on a high throughput, high precision, and a high sampling rate, or performs wireless transmission, analog signal preprocessing and/or digital signal preprocessing can also be performed on the sensing end, and a function signal can be extracted from an original bioelectrical signal (an analog electrical signal converted from a neural signal), thereby reducing a communication bandwidth and power consumption pressure, and implementing integrated sensing and computing.

S604: The signal processing apparatus 200 obtains a downstream feedback signal.

The downstream feedback signal is a signal fed back by a downstream apparatus (a downstream device) of the signal processing apparatus 200. The downstream feedback signal may include a feedback signal of the decoding apparatus 300 or a feedback signal of the effector 400. The feedback signal represents a feedback on a historical signal before the first signal.

In some possible implementations, the first signal may be a signal acquired from the brain tissue at first time, a second signal may be a signal acquired from the brain tissue at second time, and the second time is earlier than the first time. Correspondingly, the feedback signal represents a feedback on the second signal. Based on this, the feedback signal may include a brain command or a brain status that is at the second time and that is obtained by decoding the second signal by the decoding apparatus 300, or the feedback signal includes an action command or an action status that is output by the effector 400 in response to the brain command or the brain status that is at the second time. The action status may be visual information or pose information. The action command and action status may vary with an application scenario (for example, vary with a peripheral that is controlled).

It should be noted that the first time and the second time may be moments or time sequences (time periods or control periodicities). This is not limited in embodiments of this application.

S606: The signal processing apparatus 200 processes the first signal based on the feedback signal.

Specifically, the signal processing apparatus 200 may process the first signal periodically. Alternatively, when determining, based on the feedback signal, that a trigger condition is met, the signal processing apparatus 200 may process the first signal according to a processing policy corresponding to the trigger condition. The trigger condition may be a condition related to a monitoring indicator. The monitoring indicator may be set based on a service requirement. For example, the monitoring indicator may include decoding precision. The decoding precision may be determined based on the brain command or the brain status that is at the second time and that is obtained through decoding and a target brain command or a target brain status.

For example, when the decoding precision decreases sharply, which may be caused by electrode deactivation or a change in a distribution characteristic, the signal processing apparatus 200 may reselect an acquisition channel whose distribution stability meets a requirement. For another example, when the decoding precision remains a low level for a period of time, considering that an existing task may be excessively challenging/difficult for a user, the signal processing apparatus 200 may re-determine a task parameter and adjust difficulty of the task. Based on this, the trigger condition may include that the decoding precision meets a specified condition, and there may be a plurality of types of specified conditions. Different trigger conditions may correspond to different processing policies. The processing policy may include calibration, coordination control, data integration, screening of a stable distribution channel, or paradigm update (which is specifically task parameter update).

The signal processing apparatus 200 may store a correspondence between a trigger condition and a processing policy. When the trigger condition is met, the signal processing apparatus 200 may determine, based on the correspondence between a trigger and a processing policy, a processing policy corresponding to a trigger condition that is currently met.

It can be learned from descriptions of the foregoing content that, in the signal processing method in this application, the signal (for example, the first signal) acquired from the brain tissue may be processed based on the downstream feedback signal (for example, the feedback signal of the decoding apparatus 300), instead of directly inputting the signal into the decoding apparatus 300 for decoding, thereby ensuring long-term stability of signal encoding and decoding, avoiding deterioration or even failure, in long-term stability of signal encoding and decoding, that is caused due to signal non-stationarity, and meeting a service requirement.

The following provides example descriptions of the signal processing method in this application with reference to different application scenarios.

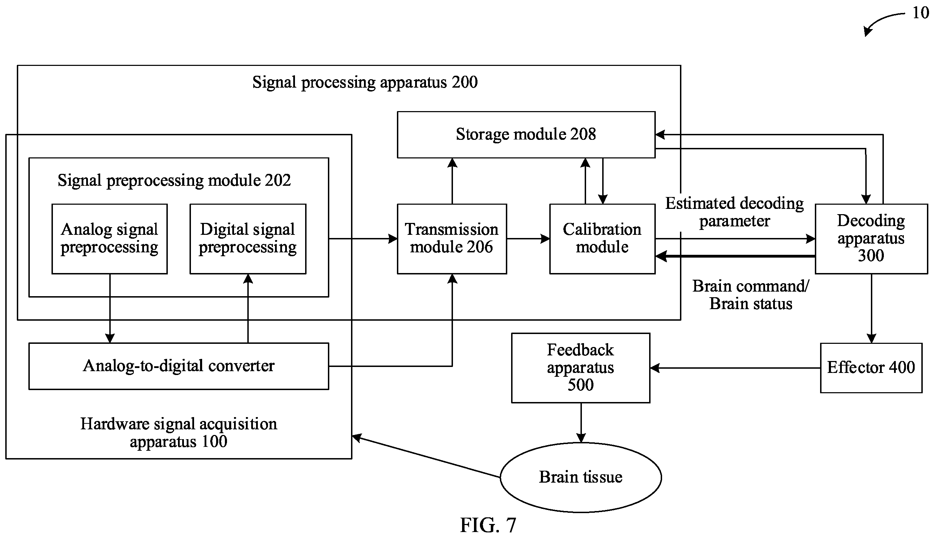

First, refer to a diagram of a scenario of a signal processing method in FIG. 7. As shown in FIG. 7, a post-processing module 204 in a signal processing apparatus 200 may include a calibration (Calibration) module, for example, a regular calibration (Regular Calibration) module or an adaptive calibration (Adaptive Calibration) module.

In this scenario, a brain-computer interface system 10 is configured to implement a brain-controlled peripheral. A brain command or a brain status obtained by a decoding apparatus 300 through decoding may be fed back to the calibration module. The following describes a process in which the calibration module performs regular calibration or adaptive calibration.

When performing regular calibration, the calibration module may obtain, at a specified frequency (or time interval), the brain command or the brain status fed back by the decoding apparatus 300, and determine decoding precision (or error) based on the brain command or the brain status fed back by the decoding apparatus 300 and a locally stored target brain status or target brain command (for example, a target brain status or brain command stored in a storage module 208). Then, the calibration module may update, based on the decoding precision, a decoding parameter (for example, a parameter of a decoding model) for decoding a first signal. During specific implementation, the calibration module may estimate a decoding parameter based on the decoding precision, and send the estimated decoding parameter (estimated decoding parameters) as an updated decoding parameter to the decoding apparatus 300, so as to update the decoding parameter.

Further, the calibration module may adjust, based on the decoding precision, the frequency or time interval for regular calibration. For example, if the decoding precision remains higher than a specified value within a specified time period, the calibration module may reduce the frequency for regular calibration or increase the time interval for regular calibration. For example, when the decoding precision is continuously greater than 0.95, the calibration module may increase the time interval for regular calibration from once a day to once a week.

When performing adaptive calibration, the calibration module may obtain the brain command or the brain status fed back by the decoding apparatus 300, and determine decoding precision (or error) based on the brain command or the brain status fed back by the decoding apparatus 300 and a locally stored target brain status or target brain command. When the decoding precision is less than the specified value (or the error is greater than another specified value), the calibration module may update, based on the decoding precision (or error), the decoding parameter for decoding the first signal. The calibration module may determine an updated decoding parameter through iteration, until the decoding precision is greater than the specified value (or the error is reduced to the another specified value).

It should be noted that the first signal may be a signal acquired from a brain tissue at first time, a second signal may be a signal acquired from the brain tissue at second time, and the second time is earlier than the first time. Correspondingly, the brain command or the brain status fed back by the decoding apparatus 300 may be a brain command or a brain status that is at the second time.

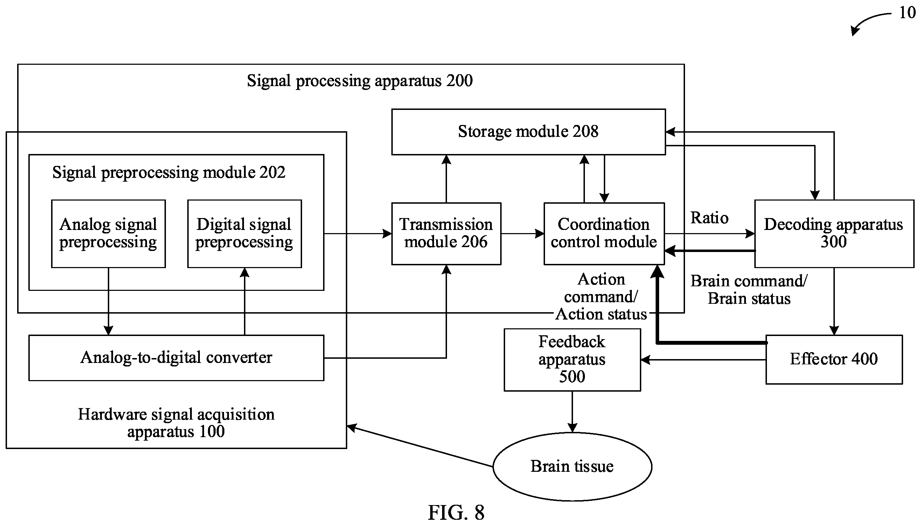

Next, refer to a diagram of a scenario of a signal processing method in FIG. 8. As shown in FIG. 8, a post-processing module 204 in a signal processing apparatus 200 may include a coordination control module, which is also referred to as a shared control module, or a shared control parameter calculation (shared control parameter calculation) module.

In this scenario, a brain-computer interface system 10 is configured to implement a brain-controlled peripheral. A brain command or a brain status obtained by a decoding apparatus 300 through decoding and an action command or an action status output by an effector 400 (for example, a peripheral) may be fed back to the coordination control module. The following describes a process in which the coordination control module performs coordination control.

Specifically, the coordination control module may obtain the brain command or the brain status fed back by the decoding apparatus 300, and determine a motion intent of a user. Specifically, the coordination control module may perform motion planning based on the brain command or the brain status, to determine the motion intent of the user. For ease of differentiation, in this application, the motion intent that is determined through motion planning and based on the brain command or the brain status obtained through decoding is denoted as a first motion intent. The coordination control module may further obtain the action status or the action command fed back by the effector 400 (for example, a peripheral like a mechanical arm). In some examples, the action status may include a pose. The coordination control module may predict a motion intent of the user based on the action command or the action status. Specifically, the coordination control module may combine a motion command or a motion status in a current control periodicity with a motion command or a motion status in a historical control periodicity stored in a storage module, and predict the motion intent of the user based on an algorithm (for example, Kalman filtering or an AI model). The motion intent predicted based on the motion command or the motion status is also referred to as a second motion intent. The coordination control module may determine a ratio of the first motion intent to the second motion intent, and deliver the ratio to the decoding apparatus 300.

The coordination control module may determine, based on a difference between the first motion intent and the second motion intent, accuracy of the motion intent that is of the user and that is obtained through decoding, and correspondingly adjust, based on the accuracy, the ratio of the first motion intent to the second motion intent.

When a time-shared coordination control mode is used for the coordination control module, the coordination control module may determine a motion intent as a final motion intent. In other words, the ratio of the first motion intent to the second motion intent is 100%:0% or 0%:100%. For example, if the difference between the first motion intent and the second motion intent is less than a preset threshold, it indicates that the accuracy is high, and the motion intent that is of the user and that is determined through decoding is reliable. The first motion intent determined through decoding may be selected, that is, the ratio of the first motion intent to the second motion intent is 100%:0%. If the difference between the first motion intent and the second motion intent is greater than the preset threshold, it indicates that the accuracy is low, and reliability of the motion intent that is of the user and that is determined through decoding is low. The predicted second motion intent may be selected, that is, the ratio of the first motion intent to the second motion intent is 0%:100%. The coordination control module delivers the ratio to the decoding apparatus 300.

When an adaptive coordination control mode is used for the coordination control module, the coordination control module may obtain a final motion intent by using a plurality of motion intents. In other words, the ratio of the first motion intent to the second motion intent is x%:(100-x)%, where a value of x is (0, 100). Specifically, the coordination control module may obtain Nash equilibrium based on paths corresponding to different motion intents and by using a dynamic decision tree. For example, the coordination control module may calculate, based on a cost-revenue function, a benefit brought by each decision (for example, each ratio), and calculate a Nash equilibrium point of the dynamic decision tree based on benefits. A ratio corresponding to the Nash equilibrium point may be used as the ratio of the first motion intent to the second motion intent. The coordination control module may deliver the ratio to the decoding apparatus 300, so that the decoding apparatus 300 performs decoding based on the ratio and controls a peripheral based on a decoding result.

It should be noted that, when performing coordination control, the coordination control module may obtain a motion intent based on a feedback signal in the current control periodicity, or may obtain a motion intent based on a feedback signal in the historical control periodicity. This is not limited in this embodiment.

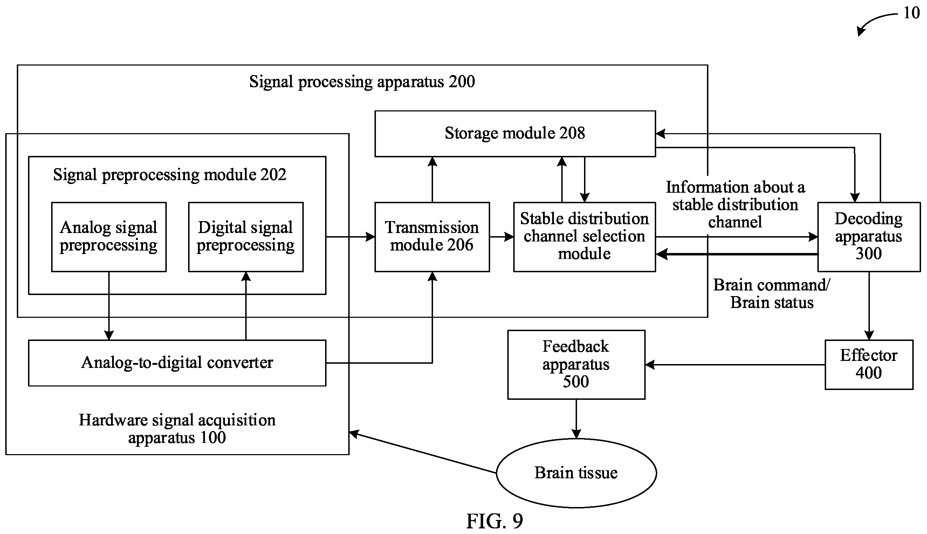

Then, refer to a diagram of a scenario of a signal processing method in FIG. 9. As shown in FIG. 9, a post-processing module 204 in a signal processing apparatus 200 may include a stable distribution channel selection (also referred to as stable channel selection, Stable Channel Selection) module.

In this scenario, a brain-computer interface system 10 is configured to implement a brain-controlled peripheral. A brain command or a brain status obtained by a decoding apparatus 300 through decoding may be fed back to the stable channel selection module. The following describes a process in which the stable distribution channel selection module performs selection of a stable distribution channel (or screening of a stable distribution channel).

During specific implementation, the stable distribution channel selection module may obtain the brain command or the brain status fed back by the decoding apparatus 300, and determine decoding precision based on the brain command or the brain status fed back by the decoding apparatus 300 and a locally stored target brain status or target brain command (for example, a target brain status or brain command stored in a storage module 208).

When the decoding precision meets a first specified condition, and the first specified condition may be that a decrease amplitude of the decoding precision within a specified time period is greater than a specified amplitude, it indicates that there is a sudden sharp drop (also referred to as a sharp decrease) in the decoding precision. Considering that the sharp decrease in the decoding precision may be caused due to electrode deactivation or a change in a distribution characteristic, the stable distribution channel selection module may determine distribution stability of a plurality of acquisition channels, and send, to the decoding apparatus 300, information (for example, Filtered Stable Signals) about an acquisition channel whose distribution stability meets a requirement, to cause the decoding apparatus 300 to decode a first signal based on the acquisition channel whose distribution stability meets the requirement.

The stable distribution channel selection module may evaluate all acquisition channels based on a standard that is set for determining distribution stability, so as to determine the acquisition channel whose stability meets the requirement, that is, a stable distribution channel. The stable distribution channel selection module selects the stable distribution channel, and transfers, to the decoding apparatus 300, information (for example, a channel identifier) about the selected stable distribution channel.

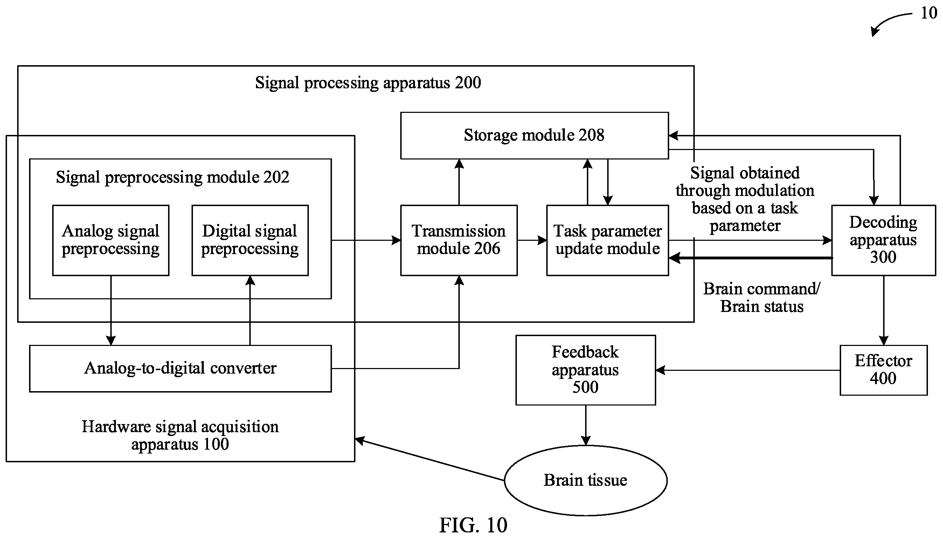

Afterwards, refer to a diagram of a scenario of a signal processing method in FIG. 10. As shown in FIG. 10, a post-processing module 204 in a signal processing apparatus 200 may include a task parameter update (task parameter update) module, which is also referred to as a paradigm update module.

In this scenario, a brain-computer interface system 10 is configured to implement a brain-controlled peripheral. A brain command or a brain status obtained by a decoding apparatus 300 through decoding may be fed back to the task parameter update module. The following describes a process in which the task parameter update module performs task parameter update (or paradigm update or paradigm optimization).

Specifically, the task parameter update module may obtain the brain command or the brain status fed back by the decoding apparatus 300, and determine decoding precision based on the brain command or the brain status fed back by the decoding apparatus 300 and a locally stored target brain status or target brain command (for example, a target brain status or brain command stored in a storage module 208).

When the decoding precision meets a second specified condition, and the second specified condition may be that the decoding precision remains lower than a precision threshold within a specified time period, it indicates that the decoding precision remains low for a period of time. A reason why the decoding precision remains a low level may be that an existing task is excessively challenging/difficult for a user. Based on this, the task parameter update module may update a task parameter, and send, to the decoding apparatus 300, a first signal (also referred to as Task Parameter-Modulated Signals) that is obtained through modulation based on an updated task parameter. A purpose of adjusting the task parameter is to adjust difficulty of the task. Control of a mechanical arm is used as an example. Adjustment of the task parameter may mean adjusting movement precision of the mechanical arm, for example, adjusting the movement precision from 1 millimeter (millimeter, mm) to 1 centimeter (centimeter, cm).

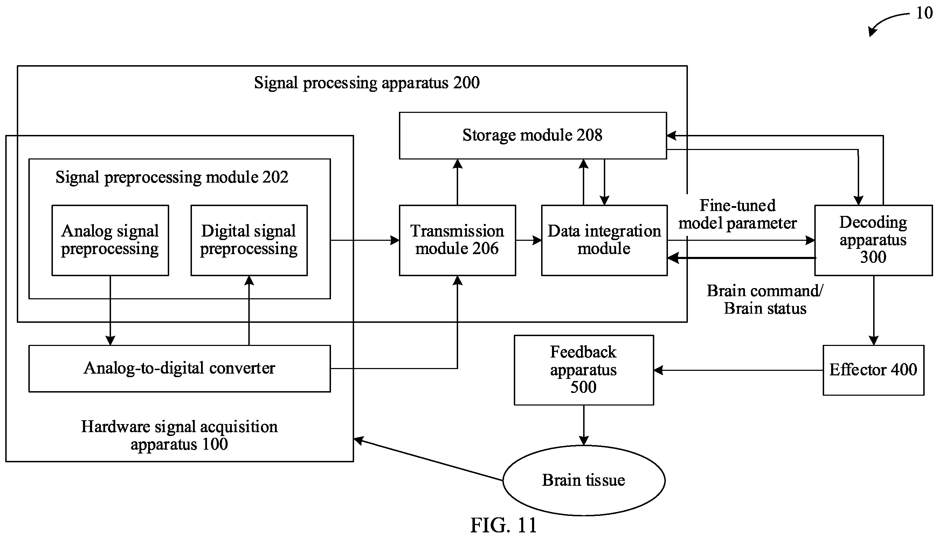

Subsequently, refer to a diagram of a scenario of a signal processing method in FIG. 11. As shown in FIG. 11, a post-processing module 204 in a signal processing apparatus 200 may include a data integration module, which is also referred to as a pre-trained model (Pre-trained Model) loading module.

In this scenario, a brain-computer interface system 10 is configured to implement a brain-controlled peripheral. A brain command or a brain status obtained by a decoding apparatus 300 through decoding may be fed back to the data integration module. The following describes a process in which the data integration module performs data integration (pre-trained model loading and fine-tuning fine-tuning).

Specifically, in an offline state, to improve generalization for decoding, the data integration module may consider integrating cross-day, cross-user (participant), and multi-modal data to pre-train a decoding model, and reserve a parameter of a pre-trained model. The parameter may include a weight of the pre-trained model. In some examples, the parameter may further include a hyperparameter of the pre-trained model, for example, an optimizer and a learning rate. During online decoding, the data integration module may obtain the brain command or the brain status fed back by the decoding apparatus 300, and determine decoding precision based on the brain command or the brain status fed back by the decoding apparatus 300 and a locally stored target brain status or target brain command (for example, a target brain status or brain command stored in a storage module 208).

When the decoding precision meets a third specified condition, and the third specified condition may be that the decoding precision remains lower than a precision threshold within a specified time period, it indicates that the decoding precision remains low for a period of time. The data integration module may consider loading the pre-trained model and performing fine-tuning based on a small amount of data on a current day. Specifically, the data integration module may perform fine-tuning on the pre-trained model by using the first signal, to obtain an updated decoding parameter, and send the updated decoding parameter to the decoding apparatus 300. The pre-trained model is obtained by pre-training the decoding model in the decoding apparatus 300 based on historical data (for example, the cross-day, cross-user, and multi-modal data).

Based on the foregoing brain-computer interface system 10, this application further provides another signal processing method. The following describes another signal processing method in this application with reference to an embodiment.

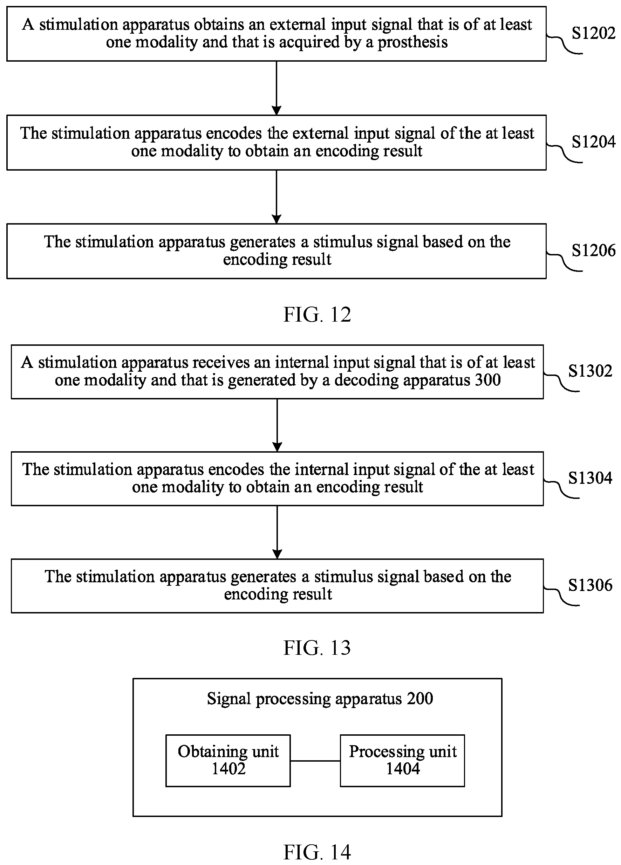

FIG. 12 is a flowchart of a signal processing method. The method may be performed by the effector 400 in the brain-computer interface system 10, and the effector 400 includes a stimulation apparatus. The method includes the following steps.

S1202: The stimulation apparatus obtains an external input signal that is of at least one modality and that is acquired by a prosthesis.

The prosthesis is also referred to as an artificial limb or a prosthetic implant (prosthetic implant), and is an artificial apparatus used to replace a body part that is lost due to a trauma, a disease, or a congenital disorder. Prostheses may be classified into different types based on body parts that are replaced, including but not limited to a visual prosthesis, an auditory prosthesis, a lower limb prosthesis, and an upper limb prosthesis. A form of the prosthesis may be a wearable device. For example, the visual prosthesis may include but is not limited to smart glasses.

A sensor of at least one modality may be deployed on the wearable device, and the modality may include but is not limited to a visual modality, an auditory modality, or a haptic modality. The wearable device is configured to acquire an external input signal of the at least one modality via the sensor of the at least one modality. An example in which the prosthesis is the smart glasses is used for description. The smart glasses may be deployed with an image sensor (for example, a camera). The image sensor may acquire an environment image, and recognize the environment image by using an image recognition model, to obtain obstacle information or traffic light status information. The stimulation apparatus may obtain the obstacle information or the traffic light status information. Based on this, the external input signal of the at least one modality may include the obstacle information or the traffic light status information. The obstacle information includes a size of an obstacle, a location of the obstacle, and a type of the obstacle (fixed or movable). When the type of the obstacle is that the obstacle is movable, the obstacle information may further include a moving speed and a moving direction. The traffic light status information may include a traffic light color and wait time for a traffic light change.

S1204: The stimulation apparatus encodes the external input signal of the at least one modality to obtain an encoding result.

The encoding result includes a parameter of a stimulation mode matching the modality. The stimulation mode includes but is not limited to a light stimulation mode, an electrical stimulation mode, and an ultrasonic stimulation mode. The stimulation apparatus may store a correspondence between a modality and a stimulation mode. When obtaining the external input signal of the at least one modality, the stimulation apparatus may determine, based on the correspondence, the stimulation mode matching the modality. For example, when the input signal is the visual modality, the stimulation mode may be the electrical stimulation mode. The stimulation apparatus may perform encoding via an encoder (encoder) based on the external input signal in a corresponding stimulation mode, to obtain the encoding result.

S1206: The stimulation apparatus generates a stimulus signal based on the encoding result.

Specifically, the stimulation apparatus may generate, based on the parameter of the stimulation mode in the encoding result, a stimulus signal corresponding to the stimulation mode. The stimulus signal may be used to stimulate a brain tissue, and the brain tissue may generate a neural signal after receiving the stimulus signal output by the stimulation apparatus, so as to implement brain sensing.

A smart glasses scenario is still used as an example for description. The stimulation apparatus generates a stimulus signal based on the encoding result, and the stimulus signal may stimulate a region responsible for vision in the brain tissue, so that a blind person wearing the smart glasses can sense a location and a size of an obstacle or sense a traffic light status, so as to avoid the obstacle or proceed when a traffic light is green.

In the embodiment shown in FIG. 12, an example in which the stimulation apparatus independently generates the stimulus signal is used for description. In another possible implementation of this application, the stimulation apparatus may generate the stimulus signal based on acquired information. The following provides descriptions with reference to another embodiment.

Refer to a flowchart of a signal processing method in FIG. 13. The method may be performed by an effector 400 in a brain-computer interface system 10, and the effector 400 includes a stimulation apparatus. The method includes the following steps.

S1302: The stimulation apparatus receives an internal input signal that is of at least one modality and that is generated by a decoding apparatus 300.

Specifically, when obtaining a specified brain status or brain command through decoding, the decoding apparatus 300 may generate the internal input signal of the at least one modality. For example, the brain status may be a state in which a user is about to experience epilepsy, and the decoding apparatus 300 may generate an internal input signal for inhibiting the epilepsy.

S1304: The stimulation apparatus encodes the internal input signal of the at least one modality to obtain an encoding result.

The encoding result includes a parameter of a stimulation mode matching the modality. The stimulation mode includes but is not limited to a light stimulation mode, an electrical stimulation mode, and an ultrasonic stimulation mode. The stimulation apparatus may store a correspondence between a modality and a stimulation mode. When obtaining the internal input signal of the at least one modality, the stimulation apparatus may determine, based on the correspondence, the stimulation mode matching the modality.

S1306: The stimulation apparatus generates a stimulus signal based on the encoding result.

The stimulation apparatus may generate, based on the parameter of the stimulation mode in the encoding result, a stimulus signal corresponding to the stimulation mode. The stimulus signal may be used to stimulate a brain tissue. The brain tissue may generate a neural signal after receiving the stimulus signal output by the stimulation apparatus, and brain control may be implemented based on the neural signal. For example, the stimulus signal may stimulate a region responsible for motion in the brain tissue, to inhibit the epilepsy for the user.

Based on the foregoing method embodiments, this application further provides a signal processing apparatus 200. As shown in FIG. 14, the apparatus 200 includes:
an obtaining unit 1402, configured to: obtain a first signal, where the first signal is a signal acquired from a brain tissue, and obtain a downstream feedback signal; and
a processing unit 1404, configured to process the first signal based on the feedback signal.

For example, the obtaining unit 1402 and the processing unit 1404 may be implemented by using software. When software is used for implementation, the obtaining unit 1402 and the processing unit 1404 may be application programs that are run on a compute device. Such an application program may be provided as a virtualization service for a user to use. The virtualization service may include a virtual machine (virtual machine, VM) service, a bare metal server (bare metal server, BMS) service, and a container (container) service. The VM service may be a service in which a virtualization technology is used to virtualize a virtual machine (virtual machine, VM) resource pool on a plurality of physical hosts (for example, compute devices), to provide, based on a need, a VM for the user to use. The BMS service is a service in which a BMS resource pool is virtualized on a plurality of physical hosts, to provide, based on a need, a BMS for the user to use. The container service is a service in which a container resource pool is virtualized on a plurality of physical hosts, to provide, based on a need, a container for the user to use. A VM is a simulated virtual computer, that is, a computer from a perspective of logic. A BMS is a scalable high-performance computing service whose computing performance is the same as that of a conventional physical machine, and features secure physical isolation. A container is a kernel virtualization technology, and may provide light-weight virtualization to isolate user space, a process, and a resource. It should be understood that the VM service, the BMS service, and the container service in the virtualization service are merely specific examples. During actual application, the virtualization service may alternatively be another light-weight or heavy-weight virtualization service. This is not specifically limited herein.

The obtaining unit 1402 and the processing unit 1404 may alternatively be implemented by using hardware. When hardware is used for implementation, the obtaining unit 1402 may be implemented through a communication interface like a transceiver. The processing unit 1404 may be a processor, or a device implemented by using an application-specific integrated circuit (application-specific integrated circuit, ASIC) or a programmable logic device (programmable logic device, PLD), or the like. The PLD may be implemented by using a complex programmable logic device (complex programmable logic device, CPLD), a field-programmable gate array (field programmable gate array, FPGA), a generic array logic (generic array logic, GAL), or any combination thereof.

In some possible implementations, the downstream feedback signal includes a feedback signal of a decoding apparatus or a feedback signal of an effector.

In some possible implementations, the first signal is a signal acquired from the brain tissue at first time, the feedback signal represents a feedback on a second signal, the second signal is a signal acquired from the brain tissue at second time, and the second time is earlier than the first time.

In some possible implementations, the feedback signal includes a brain command or a brain status that is at the second time and that is obtained by decoding the second signal by the decoding apparatus, or the feedback signal includes an action command or an action status that is output by the effector in response to the brain command or the brain status that is at the second time.

In some possible implementations, the processing unit 1404 is specifically configured to:
process, when determining, based on the feedback signal, that a trigger condition is met, the first signal according to a processing policy corresponding to the trigger condition.

In some possible implementations, the processing policy includes at least one of the following:
calibration, coordination control, data integration, screening of a stable distribution channel, or paradigm update.

In some possible implementations, the feedback signal includes a brain command or a brain status, and the processing unit 1404 is specifically configured to:
determine decoding precision based on the brain command or the brain status in the feedback signal and a target brain command or a target brain status; and
update, based on the decoding precision, a decoding parameter for decoding the first signal.

In some possible implementations, the feedback signal includes at least one of a brain command or a brain status and at least one of an action command or an action status, and the processing unit 1404 is specifically configured to:
determine a first motion intent of the user based on the brain command or the brain status, and predict a second motion intent of the user based on the action command or the action status; and
determine a ratio of the first motion intent to the second motion intent, and send the ratio to the decoding apparatus.

In some possible implementations, the feedback signal includes the brain command or the brain status that is at the second time, and the processing unit 1404 is specifically configured to:
determine decoding precision based on the brain command or the brain status in the feedback signal and a target brain command or a target brain status; and
when the decoding precision meets a first specified condition, determine distribution stability of a plurality of acquisition channels, and send, to the decoding apparatus, information about an acquisition channel whose distribution stability meets a requirement, to cause the decoding apparatus to decode the first signal based on the acquisition channel whose distribution stability meets the requirement.

In some possible implementations, the feedback signal includes a brain command or a brain status, and the processing unit 1404 is specifically configured to:
determine decoding precision based on the brain command or the brain status in the feedback signal and a target brain command or a target brain status; and
when the decoding precision meets a second specified condition, update a task parameter, and send, to the decoding apparatus, a first signal obtained through modulation based on an updated task parameter.

In some possible implementations, the feedback signal includes a brain command or a brain status, and the processing unit 1404 is specifically configured to:
determine decoding precision based on the brain command or the brain status in the feedback signal and a target brain command or a target brain status; and
when the decoding precision meets a third specified condition, perform fine-tuning on a pre-trained model by using the first signal, to obtain an updated decoding parameter, and send the updated decoding parameter to the decoding apparatus, where the pre-trained model is obtained by pre-training a decoding model in the decoding apparatus based on historical data.

In some possible implementations, the first signal is a digital signal, and the obtaining unit 1402 is specifically configured to:
obtain a neural signal acquired from the brain tissue;
convert the neural signal into an analog signal; and
convert the analog signal into the digital signal through analog-to-digital conversion.

In some possible implementations, the processing unit 1404 is further configured to:
preprocess the analog signal or the digital signal via a signal preprocessing module, to implement signal compression.

In some possible implementations, a signal preprocessing module 202 is integrated into or deployed on a hardware signal acquisition apparatus 100, and the processing unit 1404 is specifically configured to:
preprocess the analog signal or the digital signal on a side of the hardware signal acquisition apparatus 100 via the signal preprocessing module 202.

In some possible implementations, the neural signal includes a signal generated by the brain tissue by receiving a stimulus signal output by a stimulation apparatus, the stimulus signal is generated based on a result of encoding an external input signal that is of at least one modality and that is acquired by a prosthesis, and the encoding result includes a parameter of a stimulation mode matching the modality.

In some possible implementations, the prosthesis is a wearable device, a sensor of at least one modality is deployed on the wearable device, and the wearable device is configured to acquire the external input signal of at least one modality via the sensor of at least one modality.

In some possible implementations, the neural signal includes a signal generated by the brain tissue by receiving a stimulus signal output by a stimulation apparatus, the stimulus signal is generated based on a result of encoding an internal input signal that is of at least one modality and that is generated when the decoding apparatus obtains a specified brain status or brain command through decoding, and the encoding result includes a parameter of a stimulation mode matching the modality.

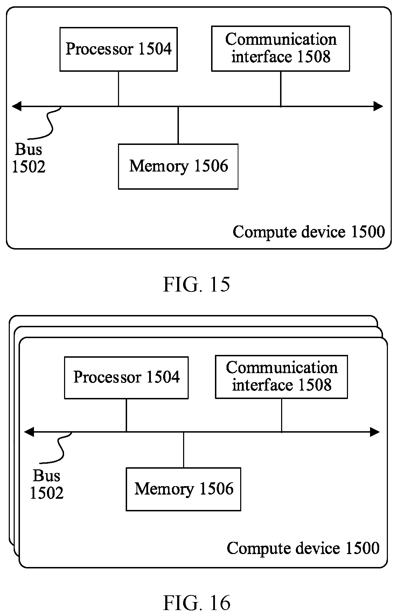

This application further provides a compute device 1500. As shown in FIG. 15, the compute device 1500 includes a bus 1502, a processor 1504, a memory 1506, and a communication interface 1508. The processor 1504, the memory 1506, and the communication interface 1508 communicate with each other through the bus 1502. The compute device 1500 may be a server or a terminal device. It should be understood that a quantity of processors and a quantity of memories in the compute device 1500 are not limited in this application.

The bus 1502 may be a peripheral component interconnect (peripheral component interconnect, PCI) bus, an extended industry standard architecture (extended industry standard architecture, EISA) bus, or the like. Buses may be classified into an address bus, a data bus, a control bus, and the like. For ease of representation, only one line is used to represent the bus in FIG. 15, but this does not mean that there is only one bus or only one type of bus. The bus 1502 may include a path for transmitting information between components (for example, the memory 1506, the processor 1504, and the communication interface 1508) of the compute device 1500.

The processor 1504 may include any one or more of processors such as a central processing unit (central processing unit, CPU), a graphics processing unit (graphics processing unit, GPU), a micro processor (micro processor, MP), or a digital signal processor (digital signal processor, DSP).

The memory 1506 may include a volatile memory (volatile memory), for example, a random access memory (random access memory, RAM). The memory 1506 may further include a non-volatile memory (non-volatile memory), for example, a read-only memory (read-only memory, ROM), a flash memory, a hard disk drive (hard disk drive, HDD), or a solid-state drive (solid-state drive, SSD). The memory 1506 stores executable program code, and the processor 1504 executes the executable program code to implement the foregoing signal processing method. Specifically, the memory 1506 stores instructions used by a signal processing apparatus 200 or a brain-computer interface system 10 to perform the signal processing method.

The communication interface 1508 is a transceiver module, for example, but not limited to, a network interface card or a transceiver, to implement communication between the compute device 1500 and another device or a communication network.

An embodiment of this application further provides a compute device cluster. The compute device cluster includes at least one compute device. The compute device may be a server, for example, a central server, an edge server, or a local server in a local data center. In some embodiments, the compute device may alternatively be a terminal device, for example, a desktop computer, a notebook computer, or a smartphone.

As shown in FIG. 16, the compute device cluster includes at least one compute device 1500. A memory 1506 in one or more compute devices 1500 in the compute device cluster may store same instructions used by a signal processing apparatus 200 or a brain-computer interface system 10 to perform the signal processing method.

In some possible implementations, the one or more compute devices 1500 in the compute device cluster may alternatively be configured to execute some instructions used by the signal processing apparatus 200 or the brain-computer interface system 10 to perform the signal processing method. In other words, the one or more compute devices 1500 may be combined to jointly execute instructions used by the signal processing apparatus 200 or the brain-computer interface system 10 to perform the signal processing method.

It should be noted that memories 1506 in different compute devices 1500 in the compute device cluster may store different instructions, to perform some functions of the signal processing apparatus 200 or the brain-computer interface system 10.

This application further provides a chip. The chip may include a processor and a communication interface. The communication interface is configured to obtain a first signal, the first signal is a signal acquired from a brain tissue, and the processor is configured to execute computer-readable instructions to perform the foregoing signal processing method.

An embodiment of this application further provides a computer-readable storage medium. The computer-readable storage medium may be any usable medium that can be stored by a compute device, or a data storage device, for example, a data center, including one or more usable media. The usable medium may be a magnetic medium (for example, a floppy disk, a hard disk, or a magnetic tape), an optical medium (for example, a DVD), a semiconductor medium (for example, a solid-state drive), or the like. The computer-readable storage medium includes instructions, and the instructions instruct the compute device to perform the foregoing signal processing method that is performed by the signal processing apparatus 200 or the brain-computer interface system 10.

An embodiment of this application further provides a computer program product including instructions. The computer program product may be software or a program product that includes the instructions and that can run on a compute device or can be stored in any usable medium. When the computer program product runs on at least one compute device, the at least one compute device is caused to perform the foregoing signal processing method.

Finally, it should be noted that the foregoing embodiments are merely intended to describe the technical solutions of the present invention but not intended to limit the present invention. Although the present invention is described in detail with reference to the foregoing embodiments, persons of ordinary skill in the art should understand that they may still make modifications to the technical solutions described in the foregoing embodiments or make equivalent replacements to some technical features thereof. Such modifications or replacements do not cause the essence of the corresponding technical solutions to depart from the protection scope of the technical solutions of embodiments of the present invention.

## Claims

1. A signal processing method, wherein the method comprises:
obtaining a first signal, wherein the first signal is a signal acquired from a brain tissue, and obtaining a downstream feedback signal; and
processing the first signal based on the feedback signal.

2. The method according to claim 1, wherein the downstream feedback signal comprises a feedback signal of a decoding apparatus or a feedback signal of an effector.

3. The method according to claim 1 or 2, wherein the first signal is a signal acquired from the brain tissue at first time, the feedback signal represents a feedback on a second signal, the second signal is a signal acquired from the brain tissue at second time, and the second time is earlier than the first time.

4. The method according to claim 3, wherein the feedback signal comprises a brain command or a brain status that is at the second time and that is obtained by decoding the second signal by the decoding apparatus, or the feedback signal comprises an action command or an action status that is output by the effector in response to the brain command or the brain status that is at the second time.

5. The method according to any one of claims 1 to 4, wherein the processing the first signal based on the feedback signal comprises:
processing, when determining, based on the feedback signal, that a trigger condition is met, the first signal according to a processing policy corresponding to the trigger condition.

6. The method according to claim 5, wherein the processing policy comprises at least one of the following:
calibration, coordination control, data integration, screening of a stable distribution channel, or paradigm update.

7. The method according to claim 5, wherein the feedback signal comprises the brain command or the brain status, and the processing the first signal according to the processing policy corresponding to the trigger condition comprises:
determining decoding precision based on the brain command or the brain status in the feedback signal and a target brain command or a target brain status; and
updating, based on the decoding precision, a decoding parameter for decoding the first signal.

8. The method according to claim 5, wherein the feedback signal comprises at least one of the brain command or the brain status and at least one of the action command or the action status, and the processing the first signal according to the processing policy corresponding to the trigger condition comprises:
determining a first motion intent of a user based on the brain command or the brain status, and predicting a second motion intent of the user based on the action command or the action status; and
determining a ratio of the first motion intent to the second motion intent, and sending the ratio to the decoding apparatus.

9. The method according to claim 5, wherein the feedback signal comprises the brain command or the brain status, and the processing, when determining, based on the feedback signal, that the trigger condition is met, the first signal according to the processing policy corresponding to the trigger condition comprises:
determining decoding precision based on the brain command or the brain status in the feedback signal and a target brain command or a target brain status; and
when the decoding precision meets a first specified condition, determining distribution stability of a plurality of acquisition channels, and sending, to the decoding apparatus, information about an acquisition channel whose distribution stability meets a requirement, to cause the decoding apparatus to decode the first signal based on the acquisition channel whose distribution stability meets the requirement.

10. The method according to claim 5, wherein the feedback signal comprises the brain command or the brain status, and the processing, when determining, based on the feedback signal, that the trigger condition is met, the first signal according to the processing policy corresponding to the trigger condition comprises:
determining decoding precision based on the brain command or the brain status in the feedback signal and a target brain command or a target brain status; and
when the decoding precision meets a second specified condition, updating a task parameter, and sending, to the decoding apparatus, a first signal obtained through modulation based on an updated task parameter.

11. The method according to claim 5, wherein the feedback signal comprises the brain command or the brain status, and the processing, when determining, based on the feedback signal, that the trigger condition is met, the first signal according to the processing policy corresponding to the trigger condition comprises:
determining decoding precision based on the brain command or the brain status in the feedback signal and a target brain command or a target brain status; and
when the decoding precision meets a third specified condition, performing fine-tuning on a pre-trained model by using the first signal, to obtain an updated decoding parameter, and sending the updated decoding parameter to the decoding apparatus, wherein the pre-trained model is obtained by pre-training a decoding model in the decoding apparatus based on historical data.

12. The method according to any one of claims 1 to 11, wherein the first signal is a digital signal, and the obtaining the first signal comprises:
obtaining a neural signal acquired from the brain tissue;
converting the neural signal into an analog signal; and
converting the analog signal into the digital signal through analog-to-digital conversion.

13. The method according to claim 12, wherein the method further comprises:
preprocessing the analog signal or the digital signal, to implement signal compression.

14. The method according to claim 13, wherein the preprocessing the analog signal or the digital signal comprises:
preprocessing the analog signal or the digital signal on a hardware signal acquisition apparatus side.

15. The method according to any one of claims 12 to 14, wherein the neural signal comprises a signal generated by the brain tissue by receiving a stimulus signal output by a stimulation apparatus, the stimulus signal is generated based on a result of encoding an external input signal that is of at least one modality and that is acquired by a prosthesis, and the encoding result comprises a parameter of a stimulation mode matching the modality.

16. The method according to claim 15, wherein the prosthesis is a wearable device, a sensor of at least one modality is deployed on the wearable device, and the wearable device is configured to acquire the external input signal of the at least one modality via the sensor of the at least one modality.

17. The method according to any one of claims 12 to 14, wherein the neural signal comprises a signal generated by the brain tissue by receiving a stimulus signal output by a stimulation apparatus, the stimulus signal is generated based on a result of encoding an internal input signal that is of at least one modality and that is generated when the decoding apparatus obtains a specified brain status or brain command through decoding, and the encoding result comprises a parameter of a stimulation mode matching the modality.

18. A signal processing apparatus, wherein the apparatus comprises:
an obtaining unit, configured to: obtain a first signal, wherein the first signal is a signal acquired from a brain tissue, and obtain a downstream feedback signal; and
a processing unit, configured to process the first signal based on the feedback signal.

19. The apparatus according to claim 18, wherein the downstream feedback signal comprises a feedback signal of a decoding apparatus or a feedback signal of an effector.

20. The apparatus according to claim 18 or 19, wherein the first signal is a signal acquired from the brain tissue at first time, the feedback signal represents a feedback on a second signal, the second signal is a signal acquired from the brain tissue at second time, and the second time is earlier than the first time.

21. The apparatus according to claim 18, wherein the feedback signal comprises a brain command or a brain status that is at the second time and that is obtained by decoding the second signal by the decoding apparatus, or the feedback signal comprises an action command or an action status that is output by the effector in response to the brain command or the brain status that is at the second time.

22. The apparatus according to any one of claims 18 to 20, wherein the processing unit is specifically configured to:
process, when determining, based on the feedback signal, that a trigger condition is met, the first signal according to a processing policy corresponding to the trigger condition.

23. The apparatus according to claim 22, wherein the processing policy comprises at least one of the following:
calibration, coordination control, data integration, screening of a stable distribution channel, or paradigm update.

24. The apparatus according to claim 22, wherein the feedback signal comprises a brain command or a brain status, and the processing unit is specifically configured to:
determine decoding precision based on the brain command or the brain status in the feedback signal and a target brain command or a target brain status; and
update, based on the decoding precision, a decoding parameter for decoding the first signal.

25. The apparatus according to claim 22, wherein the feedback signal comprises at least one of a brain command or a brain status and at least one of an action command or an action status, and the processing unit is specifically configured to:
determine a first motion intent of a user based on the brain command or the brain status, and predict a second motion intent of the user based on the action command or the action status; and
determine a ratio of the first motion intent to the second motion intent, and send the ratio to the decoding apparatus.

26. The apparatus according to claim 22, wherein the feedback signal comprises a brain command or a brain status that is at the second time, and the processing unit is specifically configured to:
determine decoding precision based on the brain command or the brain status in the feedback signal and a target brain command or a target brain status; and
when the decoding precision meets a first specified condition, determine distribution stability of a plurality of acquisition channels, and send, to the decoding apparatus, information about an acquisition channel whose distribution stability meets a requirement, to cause the decoding apparatus to decode the first signal based on the acquisition channel whose distribution stability meets the requirement.

27. The apparatus according to claim 22, wherein the feedback signal comprises a brain command or a brain status, and the processing unit is specifically configured to:
determine decoding precision based on the brain command or the brain status in the feedback signal and a target brain command or a target brain status; and
when the decoding precision meets a second specified condition, update a task parameter, and send, to the decoding apparatus, a first signal obtained through modulation based on an updated task parameter.

28. The apparatus according to claim 22, wherein the feedback signal comprises a brain command or a brain status, and the processing unit is specifically configured to:
determine decoding precision based on the brain command or the brain status in the feedback signal and a target brain command or a target brain status; and
when the decoding precision meets a third specified condition, perform fine-tuning on a pre-trained model by using the first signal, to obtain an updated decoding parameter, and send the updated decoding parameter to the decoding apparatus, wherein the pre-trained model is obtained by pre-training a decoding model in the decoding apparatus based on historical data.

29. The apparatus according to any one of claims 18 to 28, wherein the first signal is a digital signal, and the obtaining unit is specifically configured to:
obtain a neural signal acquired from the brain tissue;
convert the neural signal into an analog signal; and
convert the analog signal into the digital signal through analog-to-digital conversion.

30. The apparatus according to claim 29, wherein the processing unit is further configured to:
preprocess the analog signal or the digital signal via a signal preprocessing module, to implement signal compression.

31. The apparatus according to claim 30, wherein the signal preprocessing module is integrated into or deployed on a hardware signal acquisition apparatus, and the processing unit is specifically configured to:
preprocess the analog signal or the digital signal on the hardware signal acquisition apparatus side via the signal preprocessing module.

32. The apparatus according to any one of claims 18 to 31, wherein the neural signal comprises a signal generated by the brain tissue by receiving a stimulus signal output by a stimulation apparatus, the stimulus signal is generated based on a result of encoding an external input signal that is of at least one modality and that is acquired by a prosthesis, and the encoding result comprises a parameter of a stimulation mode matching the modality.

33. The apparatus according to claim 32, wherein the prosthesis is a wearable device, a sensor of at least one modality is deployed on the wearable device, and the wearable device is configured to acquire the external input signal of the at least one modality via the sensor of the at least one modality.

34. The apparatus according to any one of claims 18 to 31, wherein the neural signal comprises a signal generated by the brain tissue by receiving a stimulus signal output by a stimulation apparatus, the stimulus signal is generated based on a result of encoding an internal input signal that is of at least one modality and that is generated when the decoding apparatus obtains a specified brain status or brain command through decoding, and the encoding result comprises a parameter of a stimulation mode matching the modality.

35. A chip, wherein the chip comprises a processor and a communication interface, the communication interface is configured to obtain a first signal, the first signal is a signal acquired from a brain tissue, and the processor is configured to execute computer-readable instructions, to perform the signal processing method according to any one of claims 1 to 17.

36. A brain-computer interface system, wherein the system comprises a signal processing apparatus and a hardware signal acquisition apparatus, and the signal processing apparatus is configured to perform the signal processing method according to any one of 1 to 17, to process a signal output by the hardware signal acquisition apparatus, and implement brain-computer interaction.

37. The system according to claim 36, wherein the signal processing apparatus comprises a signal preprocessing module, wherein the signal preprocessing module is integrated into the hardware signal acquisition apparatus for deployment, and the signal preprocessing module is configured to:
preprocess, on the hardware signal acquisition apparatus side, the signal output by the hardware signal acquisition apparatus.

38. The system according to claim 36 or 37, wherein the system further comprises:
a stimulation apparatus, configured to generate a stimulus signal based on a result of encoding an external input signal that is of at least one modality and that is acquired by a prosthesis, wherein the encoding result comprises a parameter of a stimulation mode matching the modality, and the stimulus signal is used to stimulate a brain tissue.

39. The system according to claim 36 or 37, wherein the system further comprises:
a stimulation apparatus, configured to generate a stimulus signal based on a result of encoding an internal input signal that is of at least one modality and that is generated when a decoding apparatus obtains a specified brain status or brain command through decoding, wherein the encoding result comprises a parameter of a stimulation mode matching the modality, and the stimulus signal is used to stimulate a brain tissue.
